(19) Europäisches Patentamt
European Patent Office
Office européen des brevets

(11) Numéro de publication : **0 412 015 B1**

# (12) FASCICULE DE BREVET EUROPEEN

(45) Date de publication du fascicule du brevet : **03.08.94 Bulletin 94/31**

(51) Int. Cl.$^5$ : **C07F 9/6561,** A61K 31/675

(21) Numéro de dépôt : **90402226.6**

(22) Date de dépôt : **03.08.90**

(54) **Nouveaux dérivés N-(vincristinoyl-23) et N-(noranhydro-5' vinblastinoyl-23) d'acide amino-1 méthylphosphonique, leur procédé de préparation et les compositions pharmaceutiques qui les contiennent.**

(30) Priorité : **04.08.89 FR 8910554**

(43) Date de publication de la demande : **06.02.91 Bulletin 91/06**

(45) Mention de la délivrance du brevet : **03.08.94 Bulletin 94/31**

(84) Etats contractants désignés : **AT BE CH DE DK ES FR GB GR IT LI LU NL SE**

(56) Documents cités : **EP-A- 0 318 392**

(73) Titulaire : **ADIR ET COMPAGNIE**
**1 rue Carle Hébert**
**F-92415 Courbevoie Cédex (FR)**

(72) Inventeur : **Lavielle, Gilbert**
**1 avenue Lilly**
**F-78170 La Celle Saint Cloud (FR)**
Inventeur : **Hautefaye, Patrick**
**9 rue du Pré aux Moutons**
**F-77170 Servon Brie Comte Robert (FR)**
Inventeur : **Pierre, Alain**
**52 rue de Montval**
**F-78160 Marly le Roi (FR)**

EP 0 412 015 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés N-(vincristinoyl-23) et N-(noranhydro-5′ vinblastinoyl-23) d'acide amino-1 méthyl phosphonique, leur procédé de préparation et les compositions pharmaceutiques qui les contiennent.

Les alcaloïdes bisindoliques du type de la vincristine et de la navelbine (Brevet EP 010458) sont utilisés depuis longtemps en thérapeutique, principalement en chimiothérapie anticancéreuse. Toutefois, ces composés possèdent une toxicité élevée qui limite leurs utilisations. En outre, l'activité de la navelbine n'apparaît qu'à une posologie élevée.

Dans le but d'obtenir des composés ayant une toxicité moindre et une activité antitumorale plus importante, certains dérivés N-(désacétyl-O-4 vincristinoyl-23) aminés ont été préparés (Brevets BE 895262 ; BE 813168). Tout récemment la demande EP 318392 décrit des dérivés N-(vinblastinoyl-23) d'acide amino-1 méthyl phosphonique. Ces composés sont doués d'une très grande activité et ont une toxicité moindre (neurotoxicité) par rapport aux produits de référence.

Les besoins cliniques cependant, favorisent le développement constant de nouvelles molécules anticancéreuses dans le but d'obtenir une activité améliorée et une toxicité secondaire moindre.

La demanderesse a maintenant découvert que certains dérivés phosphoniques de la vincristine et de la navelbine, de structure originale, possèdent des propriétés pharmacologiques très intéressantes. En effet, les composés de la présente invention sont doués d'une activité antitumorale très supérieure à tous les dérivés de la vincristine et de la navelbine déjà connus. En plus, les toxicités observées sont significativement inférieures à celles des produits de référence.

La présente invention a plus particulièrement pour objet les dérivés d'acide amino-1 méthyl phosphonique de formule générale I:

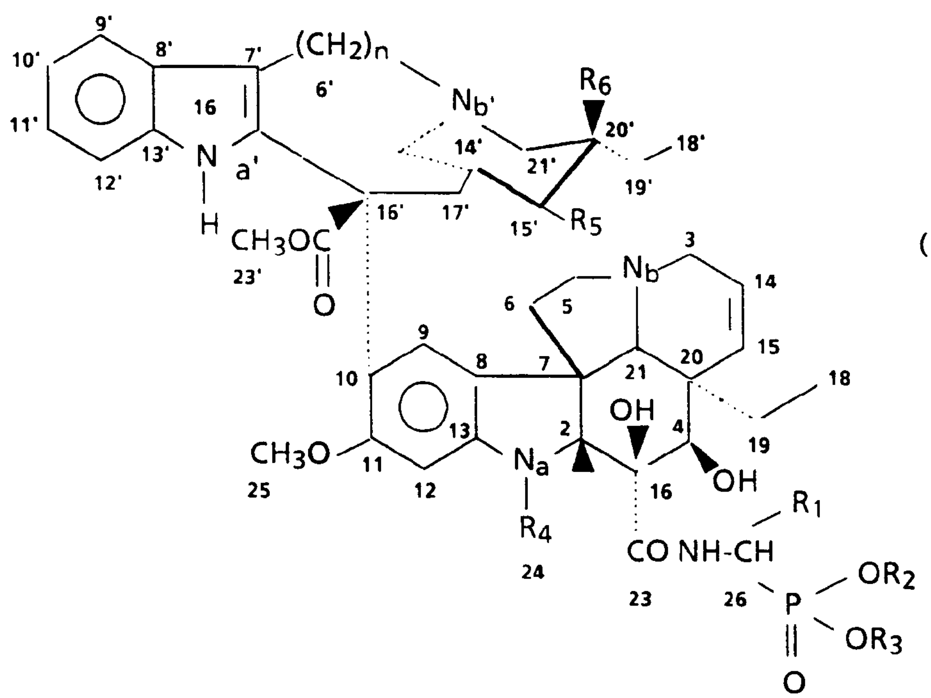

(I)

dans laquelle:

- $R_1$ représente un atome d'hydrogène, un radical alkyle linéaire ou ramifié renfermant de 1 à 6 atomes de carbone, un radical alcènyle linéaire ou ramifié renfermant de 2 à 6 atomes de carbone, un radical arylalkyle de 7 à 10 atomes de carbone pouvant porter comme substituant sur le cycle aromatique un atome d'halogène, un radical hydroxyle ou un radical alkyle ou alcoxy renfermant chacun de 1 à 5 atomes de carbone, un radical indolyl-2 méthyle, un radical imidazolyl-4 méthyle, ou un radical alocoxycarbonylméthyle renfermant de 3 à 11 atomes de carbone,
- $R_2$ et $R_3$ identiques ou différents représentent chacun un radical alkyle linéaire ou ramifié renfermant de 1 à 6 atomes de carbone,
- n est égal à 1 ou 2,
- $R_4$ représente un atome d'hydrogène, un radical formyle ou un radical méthyle, à condition toutefois que

$R_4$ ne soit jamais un radical méthyle quand n est égal à 2,
et

- $R_5$ et $R_6$ soit forment ensemble une double liaison, soit $R_5$ représente un atome d'hydrogène et $R_6$ un radical hydroxy,

sous forme de mélange de diastéréo-isomères ou d'isomères purs, leurs $N_{b'}$-oxydes et leurs sels d'addition à un acide minéral ou organique pharmaceutiquement acceptable.

La présente invention a également pour objet le procédé de préparation de composés de formule générale I, caractérisé,

soit

en ce que l'on fait réagir une amine, sous forme racémique ou optiquement pure, de formule générale II :

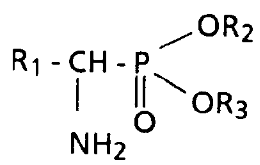

(II)

dans laquelle la définition de $R_1$, $R_2$ et $R_3$ demeure celle définie précédemment pour la formule générale I, avec un composé de formule III :

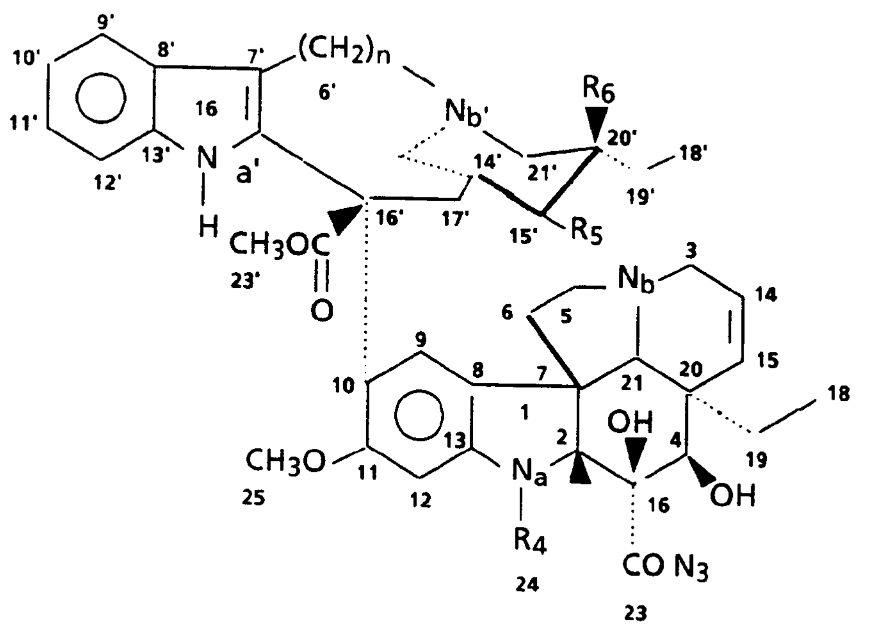

(III)

dans laquelle n, $R_5$ et $R_5$ ont la signification définie précédemment pour la formule générale I, et $R_4$ représente un atome d'hydrogène ou un radical méthyle, pour former respectivement sous forme de mélange de diastéréoisomères ou d'isomères purs les composés de formule générale I dans laquelle n, $R_1$, $R_2$, $R_3$, $R_5$ et $R_5$ ont la signification définie précédemment et $R_4$ représente un atome d'hydrogène ou un radical méthyle,

puis, pour former les composés de formule générale I dans laquelle $R_4$ représente un radical formyle, qu'on soumet les composés de formule Ia :

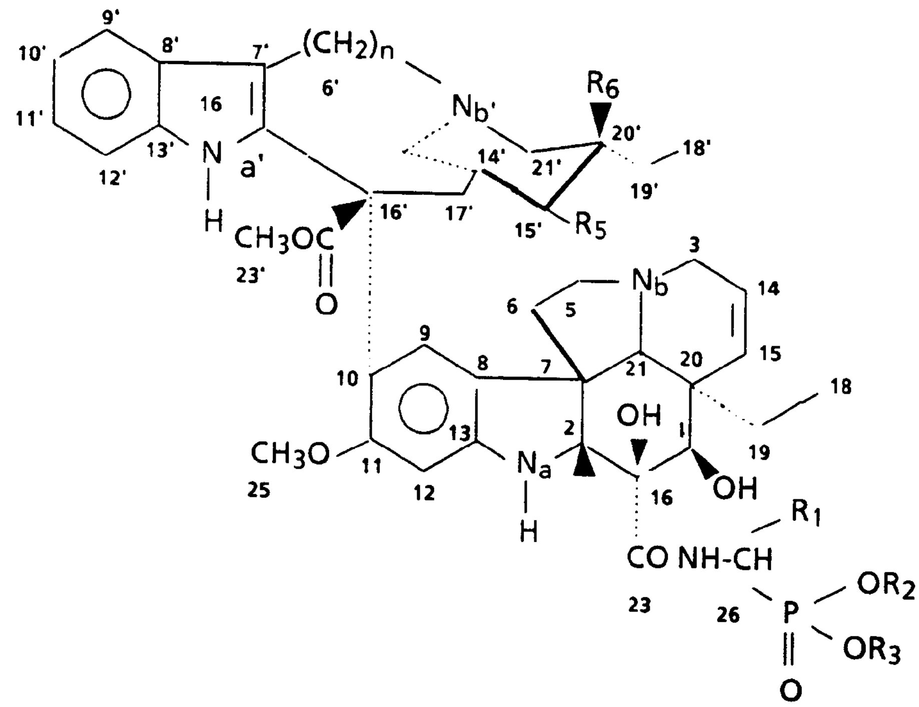

(Ia)

à l'action d'acide formique en présence d'anhydride acétique,

soit

en ce que l'on traite un composé sous forme racémique ou optiquement pur de formule IV :

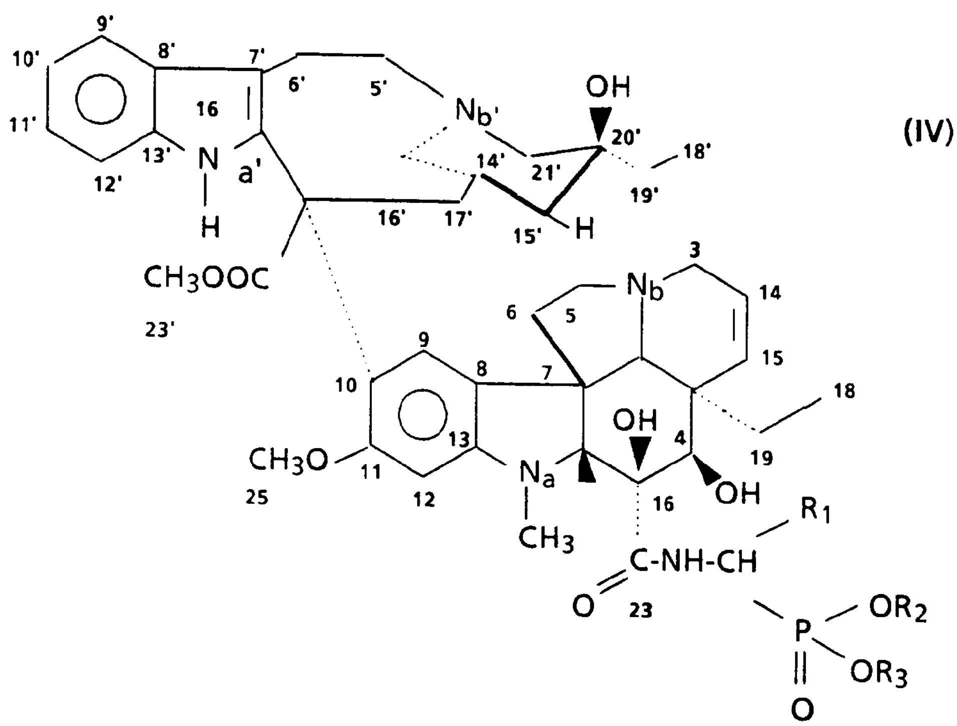

(IV)

dans laquelle la définition de $R_1$, $R_2$ et $R_3$ demeure identique à celle définie précédemment pour la formule I, par l'ion permanganate en milieu acide dans un solvant inerte, à une température comprise entre -40°C et -75°C, pour former les composés de formule générale I, dans laquelle n est égal à 2 et $R_4$ représente un radical formyle,

et ensuite,

que l'on salifie les composés de formule générale I avec un acide minéral ou organique pharmaceutiquement

acceptable,
ou
que l'on transforme aux $N_{b'}$-oxydes correspondants à l'aide d'un solvant organique basique saturé d'oxygène.

Les amino-1 méthyl phosphonates, composés de formule générale II, peuvent être préparés selon trois procédés :

- soit par réduction à l'aide de zinc des composés de formule générale V :

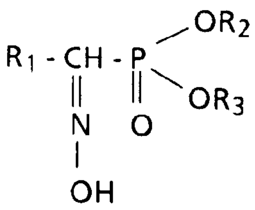

(V)

dans laquelle $R_1$, $R_2$ et $R_3$ ont la signification précédemment définie pour la formule I, en solution dans l'acide formique ;

- soit par alkylation des imines de formule générale VI :

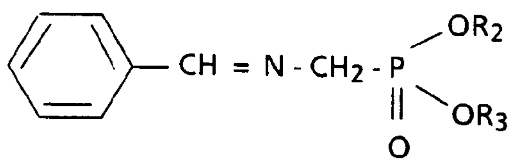

(VI)

dans laquelle $R_2$ et $R_3$ ont la signification précédemment définie pour la formule I,
à l'aide d'un halogénure d'alkyle de formule générale VII :

**$R_1$-X (VII)**

dans laquelle la définition de $R_1$ reste identique à celle donnée pour la formule I selon la méthode décrite dans Bull.Soc.Chim.Fr.(1978),II,p.95 ;

- soit par action du diphénylphosphoryle azide (D.P.P.A.) sur des acides de formule générale VIII :

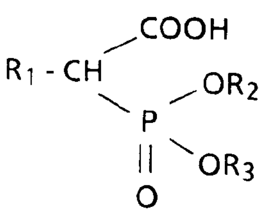

(VIII)

dans laquelle $R_1$, $R_2$, $R_3$ ont la signification précédemment définie pour la formule I,
pour former les carbamates de formule générale IX :

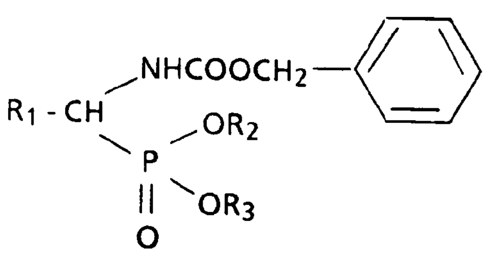

(IX)

dans laquelle la définition de $R_1$, $R_2$ et $R_3$ reste identique à celle donnée pour la formule I,
qui sont soumis ensuite à une hydrogénolyse catalytique pour former les amines de formule générale II (Tetrahedron Letters,(1983),24,(49),p.5461).

Les composés de formule générale V sont obtenus par action de l'hydroxylamine sur les cétones de for-

mule générale X :

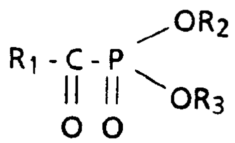

(X)

dans laquelle $R_1$, $R_2$ et $R_3$ ont la signification précédemment définie pour la formule I, selon le procédé décrit dans Synthesis,(1981),p. 57. La préparation des composés de formule générale IX est connue (Houben Weyl, Methoden der Organischen Chemie, Georg Thiem Verlag, Stuttgart, 5e ed, vol.12/1,p.453).

La préparation des composés de formule générale VI est décrite dans Tetrahedron Letters,(1973),46,p.4645.

La formylation des composés de formule Ia est effectuée selon un procédé déjà connu. (J.Org.Chem.(1958), 23,p.727).

Les composés de formule III sont préparés en deux étapes. La première consiste à additionner un excès d'hydrazine anhydre à une solution de vincristine ou de navelbine base dans le méthanol anhydre. Le composé obtenu, de formule XI :

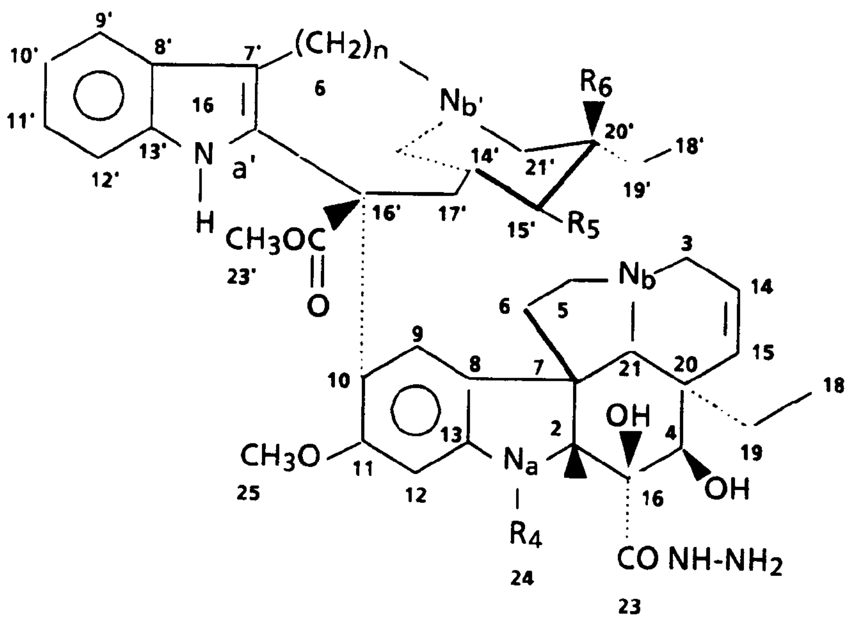

(XI)

dans laquelle n, $R_5$ et $R_6$ ont la signification précédemment définie pour la formule générale I et R4 représente un atome d'hydrogène ou un radical méthyle, est ensuite soumis à l'action du nitrite de sodium en milieu acide, pour former les composés de formule III.

L'acide utilisé lors de cette dernière réaction peut être l'acide chlorhydrique. La température du milieu réactionnel est maintenue entre 0°-5°C.

Les alcylazides formés sont extraits ensuite par un solvant aprotique non soluble à l'eau, de préférence le chlorure de méthylène. Les composés de formule III ne sont de préférence pas isolés. En effet, la solution organique les contenant est concentrée, et ensuite les composés de formule III sont mis en contact à température ambiante avec les dérivés d'acide amino-1 méthyl phosphonique de formule générale II.

Les amines de formule générale II, peuvent être obtenues optiquement pures ou par cristallisation fractionnée de leurs sels avec un acide optiquement pur, (J.Org.Chem.,(1963),28,p.2483) ou selon le procédé décrit dans Liebigs Ann.Chem.,(1987),p.45.

La préparation des composés de formule générale IV est déjà décrite dans la littérature (Demande de brevet EP 318 392), et l'oxydation de ces composés par l'ion permanganate est effectuée selon un procédé déjà connu (Demande de brevet EP 0117 861).

Les composés de formule générale I peuvent être obtenus sous forme de diastéréoisomères purs par condensation des alcylazides correspondants avec une amine de formule générale II optiquement pure ou à partir de mélange de diastéréoisomères, que l'on sépare ensuite par chromatographie liquide haute pression

(H.P.L.C.).

Les composés de la formule générale I sont des dérivés de descarbométhoxy-16 désacétyl-O-4 vincristine carboxamide-16 et de descarbométhoxy-16 désacétyl-O-4 noranhydro-5′ vinblastine carboxamide-16. Toutefois, on préfère les désigner comme étant des dérivés N-(vincristinoyl-23) d'acide amino-1 méthyl phosphonique et des dérivés N-(noranhydro-5′ vinblastinoyl-23) d'acide amino-1 méthyl phosphonique.

Les suffixes (+) ou (-) employés pour désigner certains composés n'indiquent pas le sens dans lequel ces composés tournent le plan de polarisation de la lumière. mais indiquent qu'ils sont obtenus par une amine de formule II optiquement pure ; (+) ou (-).

Parmi les acides pharmaceutiquement acceptables pour la préparation des sels d'addition aux composés de formule générale I, on peut citer les acides phosphorique, chlorhydrique, citrique, oxalique, maléïque, sulfurique, tartrique, mandélique, fumarique, méthanesulfonique etc...

Les composés selon l'invention, ainsi que leurs sels d'addition, sont doués de propriétés pharmacologiques fort intéressantes, et se distinguent des autres dérivés de la vincristine ou de la navelbine déjà connus.

Les composés de l'invention ont été testés par leur capacité à prolonger la survie de souris porteuses de cellules tumorales (P 388 et sur un cancer du poumon humain) par voie intrapéritonéale selon les protocoles recommandés par l'Institut National du Cancer USA (Geran R.I. et coll., Cancer Chemotherapy Reports,(1972),III,3,N°2,p.1-87), et reconnus comme représentatifs de l'effet antitumoral en clinique humaine (Driscoll J.C.S Cancer Treatment Reports,(1984),68,N°1,p.63-85 et "In vivo cancer Models" US Department of Health and Human Services NIH Publication N° 84-2635 Feb. 1984).

Les composés de la présente invention se sont révélés non seulement capables de ralentir la croissance des tumeurs greffées chez la souris, mais également de guérir des animaux. En effet, de nombreuses rémissions complètes ont été observées. En plus, des essais comparatifs avec des produits de références décrits dans la littérature -vinblastine, vincristine et navelbine- et avec le composé le plus actif décrit dans la demande de brevet EP 318 392, ont démontré que les composés de l'invention ont une activité très supérieure par rapport aux composés déjà connus.

Les composés de la présente invention sont utiles chez l'homme et les animaux en cas de maladie de Hodgkin, lymphomes non hodgkiniens, cancers du testicule, épithélioma du sein et de l'ovaire, sarcome de Kaposi, choriocarcinome histocytose, rhabdomyosarcomes, neuroblastomes, tumeurs de Wilms, sarcomes d'Ewing, cancer du poumon etc... D'autres applications thérapeutiques peuvent être aussi envisagées pour les composés de l'invention. En effet, il est connu que les alcaloïdes bisindoliques et leurs dérivés sont actifs pour le traitement de certaines arthrites ou de psoriasis. (Brevets US 4,208,411 et 3,749,784).

L'invention s'étend aussi aux compositions pharmaceutiques renfermant comme principe actif au moins un composé de formule générale I, un de ses isomères optiques ou un de ses sels d'addition avec un acide minéral ou organique ou un de ses $N_{b'}$-oxydes avec un ou plusieurs excipients, inertes, non toxiques et appropriés.

Les compositions pharmaceutiques ainsi obtenues sont présentées avantageusement sous des formes diverses telles que par exemple comprimés, dragées, gélules, crèmes pour applications locales, suppositoires, solutions injectables etc... Elles peuvent contenir des doses de 0,1 à 100 mg d'un ou plusieurs composés de l'invention.

Pour leur application thérapeutique, les composés de l'invention, leurs isomères optiques ou leurs sels d'addition, sont de préférence administrés par voie parentérale. D'une manière générale, les composés de l'invention peuvent être utilisés en s'inspirant des techniques et des limitations qui sont connues pour les traitements thérapeutiques avec les autres alcaloïdes de la classe des Vinca.

La posologie peut varier largement en fonction de l'âge, du poids du patient, de la nature et de la sévérité de l'affection, de la voie d'administration ainsi que du schéma thérapeutique utilisé. Les doses hebdomadaires totales s'échelonneront généralement de 0,01 à environ 20 mg/kg.

Les composés de l'invention peuvent être utilisés seuls ou en combinaison avec un ou plusieurs agents carcinostatiques incluant par exemple les agents alkylants, les antimétabolites tels que le méthotrexate, le fluoro-5-uracile, la mercapto-6 purine, la thio-6 guanine, les arabinosides de la cytosine et les antibiotiques tels que l'actinomycine D, la daunorubicine, l'adriamycine, et le cis-diamino dichloro platine etc...

Les exemples suivants, donnés à titre non-limitatif, illustrent l'invention.

Les spectres de résonance magnétique nucléaire (R.M.N.) du $^{13}C$ et du proton ont été enregistrés à 400 MHz.

**EXEMPLE 1**

**N-(désacétyl-0-4 noranhydro-5′ vinblastinoyl-23) amino-1 méthyl-2 propyl phosphonate de diéthyle**

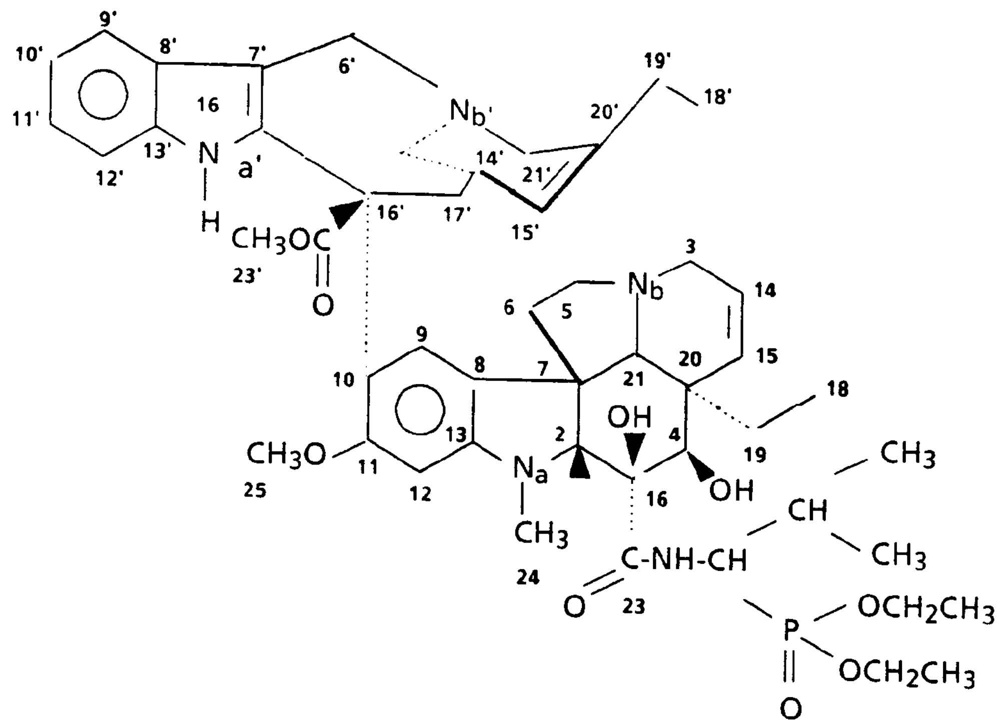

A une solution de 130 ml d'acide chlorhydrique N, refroidie à 0°C et contenant 2,34 mmoles de N-(désacétyl-0-4 noranhydro-5′ vinblastinoyl-23) carbohydrazide, on ajoute 5,20 mmoles de nitrite de sodium. Après 10 minutes de contact à 0°C, on ajuste le pH du milieu à 8,8 à l'aide d'une solution glacée et saturée de bicarbonate de sodium et on extrait rapidement à l'aide de 4 fois 100 ml de dichlorométhane. Les phases organiques réunies sont lavées à l'aide d'une solution saturée de chlorure de sodium et séchées sur sulfate de magnésium anhydre. On concentre la phase organique jusqu'à un volume de 50 ml, on ajoute 3,10 mmoles d'amino-1 méthyl-2 propyl phosphonate de diéthyle (Synthesis,(1981),57) et on abandonne le milieu réactionnel 24 heures à température ambiante.

Après évaporation du solvant, le résidu est purifié par chromatographie sur colonne de silice (230-400 Mesh) en utilisant comme éluant un mélange de toluène et d'éthanol (80V/20V).

On recueille le produit attendu que l'on cristallise dans un mélange d'éther éthylique et d'éther de pétrole (50V/50V).

Rendement : 32%

**EXEMPLE 2**

**(+) N-(désacétyl-0-4 noranhydro-5′ vinblastinoyl-23) amino-1 méthyl-2 propyl phosphonate de diéthyle**

Ce composé est préparé selon la méthode décrite ci-dessus à partir de 1,4 g de N-(désacétyl-0-4 noranhydro-5′ vinblastinoyl-23) carbohydrazide et de 0,5 g de (+) amino-1 méthyl-2 propyl phosphonate de diéthyle.

Après 24 heures d'agitation à température ambiante, le solvant est séparé pour obtenir 1,45 g de produit que l'on dissout dans 4 ml d'éthanol. Cette solution est ensuite purifiée par chromatographie en utilisant une colonne contenant 500 g de Lichroprep RP 18 (15-25 μm) On élue à l'aide d'un mélange de méthanol et de solution aqueuse d'hydrogenophosphate disodique 0,01 M (70V/30V). Le débit de la phase mobile est fixé à 20 ml/min. Les fractions 540 à 590 sont rassemblées et après condensation sous vide, le résidu est extrait par le chlorure de méthylène, puis, la phase organique est séchée sur sulfate de magnésium anhydre. Après évaporation du solvant, on obtient le (+) N-(désacétyl-O-4 noranhydro-5′ vinblastinoyl-23) amino-1 méthyl-2 propyl phosphonate de diéthyle.

Rendement : 30 %

Le sulfate correspondant est formé après addition d'une quantité adéquate d'éthanol sulfurique.

Les spectres de résonance magnétique nucléaire donnés ci-après ont été réalisés avec le sulfate de (+) N-(désacétyl-O-4 noranhydro-5′ vinblastinoyl-23) amino-1 méthyl-2 propyl phosphonate de diéthyle.

Spectre de résonance magnétique nucléaire du $^{13}C$ (solvant $D_2O$):

| | | | | | |
|---|---|---|---|---|---|
| 178,4 | ppm $CO_2$ | 82,0 | ppm C2 | 34,1 | ppm C19 |
| 175,25/175,3 | ppm CONH | 69,7 | ppm C21 | 31,4 | ppm <u>C</u>H($CH_3$)$_2$ |
| 161,1 | ppm C11 | 66,6/66,8 | ppm (O<u>C</u>$H_2CH_3$)$_2$ | 29,3 | ppm C19' |
| 156,0 | ppm C13 | 58,6 | ppm C23' | 20,7/22,5 | ppm CH(<u>C</u>$H_3$)$_2$ |
| 133,8 | ppm C13' | 57,0 | ppm C16' | 18,3 | ppm ($OCH_2$<u>C</u>$H_3$)$_2$ |
| 133,7 | ppm C15 | 56,2 | ppm C25 | 13,7 | ppm C18' |
| 130,3 | ppm C2' | 53,4/54,9 | ppm C26 | 9,7 | ppm C18 |
| 126,4 | ppm C10' | 54,8 | ppm C7 | | |
| 123,7 | ppm C14' | 52,1 | ppm C3+C5 | | |
| 123,6 | ppm C14 | 49,2 | ppm C21' | | |
| 120,4 | ppm C9' | 46,1 | ppm C3' | | |
| 114,7 | ppm C12' | 45,5 | ppm C6 | | |
| 106,9 | ppm C7' | 45,4 | ppm C20 | | |
| 97,5 | ppm C12 | 42,1 | ppm C24 | | |
| 83,7 | ppm C16 | 36,5 | ppm C17' | | |

Spectre de résonance magnétique nucléaire du proton (solvant $D_2O$) :

| | | | | | | | |
|---|---|---|---|---|---|---|---|
| 7,84 | ppm | 1H | $C^{9'}$-H | 3,73 | ppm | 1H | $C^{2}$-H |
| 7,54 | ppm | 1H | $C^{12'}$-H | 3,55 | ppm | 1H | $C^{21}$-H |
| 7,35 | ppm | 2H | $C^{10'}$-H+$C^{11'}$-H | 3,42/3,94 | ppm | 2H | $C^{3}$-H |
| 6,50 | ppm | 1H | $C^{12}$-H | 3,14/3,85 | ppm | 2H | $C^{5}$-H |
| 6,45 | ppm | 1H | $C^{9}$-H | 2,91/3,59 | ppm | 2H | $C^{3'}$-H |
| 5,98 | ppm | 1H | $C^{14}$-H | 2,87 | ppm | 3H | $C^{25}$-H |
| 5,88 | ppm | 1H | $C^{15}$-H | 2,60/3,14 | ppm | 2H | $C^{17'}$-H |
| 5,83 | ppm | 1H | $C^{15'}$-H | 2,34 | ppm | 1H | $\underline{CH}(CH_3)_2$ |
| 4,70/4,90 | ppm | 1H | $C^{21'}$-H | 2,11 | ppm | 2H | $C^{19'}$-H |
| 4,39 | ppm | 1H | $C^{26}$-H | 2,02/2,44 | ppm | 2H | $C^{6}$-H |
| 4,24 | ppm | 4H | $(O\underline{CH_2}-CH_3)_2$ | 2,02 | ppm | 1H | $C^{14'}$-H |
| 4,08 | ppm | 1H | $C^{17}$-H | 1,38 | ppm | 6H | $(OCH_2\underline{CH_3})_2$ |
| 3,93 | ppm | 3H | $C^{25}$-H | 1,32/1,74 | ppm | 2H | $C^{19}$-H |
| 3,80/4,15 | ppm | 2H | $C^{5'}$-H | 1,10 | ppm | 3H | $C^{18'}$-H |
| 3,79 | ppm | 3H | $C^{23'}$-H | 1,09/1,11 | ppm | 6H | $CH(\underline{CH_3})_2$ |
| | | | | 0,81 | ppm | 3H | $C^{18}$-H |

## EXEMPLE 3

**(-) N-(désacétyl-0-4 noranhydro-5' vinblastinoyl-23) amino-1 méthyl-2 propyl phosphonate de diéthyle**

Ce composé est obtenu selon le procédé décrit pour l'exemple 2 mais en utilisant le (-) amino-1 méthyl-2 propyl phosphonate de diéthyle.

Lors de la purification par chromatographie, les fractions 400 à 500 sont réunies et traitées comme décrit ci-dessus.

Rendement : 29 %

Spectre de résonance magnétique nucléaire du $^{13}C$ (solvant $CDCl_3$):

| | | |
|---|---|---|
| 174,2 ppm $CO_2$- | 110,6 ppm C12' | 44,8 ppm C6 |
| 172,9 ppm CONH | 93,5 ppm C12 | 44,3 ppm C3' |
| 158,1 ppm C11 | 82,9 ppm C2 | 42,9 ppm C21' |
| 152,8 ppm C13 | 80,2 ppm C16 | 42,5 ppm C20 |
| 134,3 ppm C13' | 64,6 ppm C21 | 39,1 ppm C24 |
| 134,0 ppm C20' | 62,0 ppm $(OCH_2CH_3)_2$ | 35,0 ppm C17' |
| 132,1 ppm C2' | 55,7 ppm C25 | 31,1 ppm C19 |
| 129,5 ppm C15 | 54,9 ppm C16' | 29,8 ppm $CH(CH_3)_2$ |
| 128,5 ppm C8' | 53,6 ppm C7 | 27,7 ppm C19' |
| 124,5 ppm C14 | 53,4 ppm C6' | 20,8/18,2 ppm $CH(CH_3)_2$ |
| 122,7 ppm C10' | 52,2 ppm C23' | 16,3 ppm $(OCH_2CH_3)_2$ |
| 120,0 ppm C11' | 50,3 ppm C3 | 12,0 ppm C18' |
| 118,3 ppm C9' | 49,8 ppm C26 | 8,4 ppm C18 |
| 118,2 ppm C7' | 49,0 ppm C5 | |

**EXEMPLE 4**

**(+) N-($N_a$-desformyl désacétyl-0-4 vincristinoyl-23) amino-1 méthyl-2 propylphosphonate de diéthyle**

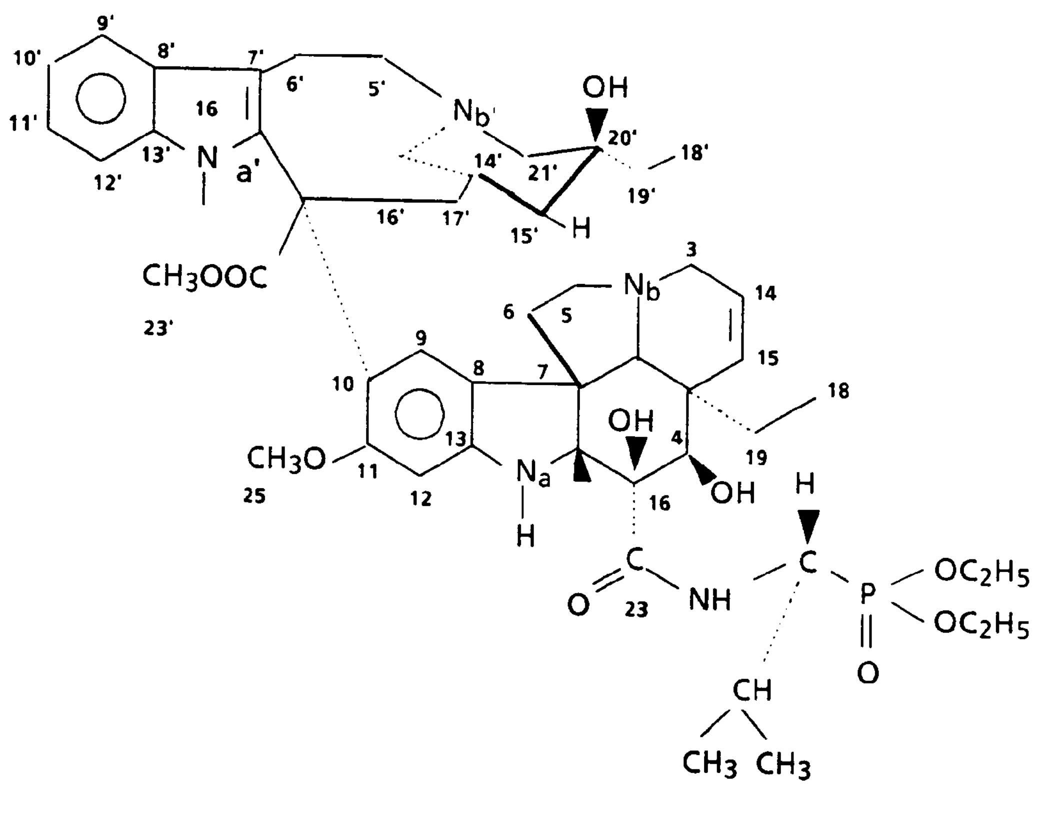

4,6 g de $N_a$-desformyl désacétyl-O-4 vincristinoyl-23 azide en solution dans 50 ml de dichlorométhane

sont agités pendant 24 heures à température ambiante en présence de 1,2 g de (+) amino-1 méthyl-2 propylphosphonate de diéthyle, pour obtenir le produit attendu. Le (+) N-($N_a$-desformyl désacétyl-O-4 vincristinoyl-23) amino-1 méthyl-2 propylphosphonate de diéthyle est ensuite purifié par chromatographie selon le procédé décrit dans l'exemple 2.
Rendement : 27 %

Spectre de résonance magnétique nucléaire du proton (solvant $CDCl_3$):

| | | | |
|---|---|---|---|
| 8,5 | ppm 1H NH | 2,80/3,30 | ppm 2H $C^5$-H |
| 8,1 | ppm 2H NH,NHCO | 2,74 | ppm 2H $C^{21'}$-H |
| 7,50 | ppm 1H $C^{9'}$-H | 2,64 | ppm 2H $C^{21}$-H |
| 7,20/7,12 | ppm 3H $C^{10'}$-H+$C^{11'}$-H+$C^{12'}$-H | 2,40/3,21 | ppm 2H $C^{3'}$-H |
| 6,65 | ppm 1H $C^9$-H | 2,45/3,10 | ppm 2H $C^3$-H |
| 6,27 | ppm 1H $C^{12}$-H | 2,35/4,03 | ppm 2H $C^{17'}$-H |
| 5,85 | ppm 1H $C^{14}$-H | 2,34 | ppm 1H $\underline{CH}(CH_3)_2$ |
| 5,70 | ppm 1H $C^{15}$-H | 1,90/2,10 | ppm 2H $C^6$-H |
| 4,42 | ppm 1H $C^{26}$-H | 1,51 | ppm 2H $C^{15'}$-H |
| 4,10 | ppm 5H $C^4$-H+$(O\underline{CH_2}CH_3)_2$ | 1,40 | ppm 2H $C^{19}$-H |
| 3,91 | ppm 1H $C^2$-H | 1,38 | ppm 6H $(OCH_2-\underline{CH_3})_2$ |
| 3,75 | ppm 3H $OCH_3$ | 1,25/1,60 | ppm 2H $C^{19'}$-H |
| 3,65 | ppm 3H $C^{23'}$-H | 1,14 | ppm 6H $CH(\underline{CH_3})_2$ |
| 3,31 | ppm 2H $C^{5'}$-H | 0,90 | ppm 6H $C^{18}$-H+$C^{18'}$-H |

**EXEMPLE 5**

**(-) N-($N_a$-desformyl désacétyl-0-4 vincristinoyl-23) amino-1 méthyl-2 propyl phosphonate de diéthyle**

Ce composé a été préparé selon le procédé décrit dans l'exemple 4 mais en utilisant le (-) amino-1 méthyl-2 propyl phosphonate de diéthyle. Le composé obtenu a été purifié par chromatographie selon la méthode décrite dans l'exemple 3.
Rendement : 29 %

Spectre de résonance magnétique nucléaire du proton (solvant $CDCl_3$):

| | |
|---|---|
| 8,7 | ppm 1H NH |
| 8,03 | ppm 1H NHCO |
| 7,48 | ppm 1H $C^{9'}$-H |
| 7,17/7,15/7,09 | ppm 3H $C^{10'}$-H+$C^{11'}$-H+$C^{12'}$-H |
| 6,63 | ppm 1H $C^9$-H |
| 6,27 | ppm 1H $C^{12}$-H |
| 5,91 | ppm 1H $C^{14}$-H |
| 5,79 | ppm 1H $C^{15}$-H |
| 4,50 | ppm 1H $C^{26}$-H |
| 4,13 | ppm 1H $C^4$-H |
| 4,08 | ppm 4H $(O\underline{CH_2}CH_3)_2$ |
| 3,91 | ppm 1H $C^2$-H |
| 3,75 | ppm 3H $OCH_3$ |
| 3,63 | ppm 3H $C^{23'}$-H |
| 3,28 | ppm,2H $C^{5'}$-H |
| 2,83/3,28 | ppm 2H $C^5$-H |
| 2,75 | ppm 2H $C^{21'}$-H |
| 2,70 | ppm 2H $C^{21}$-H |
| 2,41/3,15 | ppm 2H $C^3$-H |
| 2,35/4,00 | ppm 2H $C^{17'}$-H |
| 2,35 | ppm 1H $\underline{CH}(CH_3)_2$ |
| 1,88/2,10 | ppm 2H $C^6$-H |
| 1,54 | ppm 2H $C^{15'}$-H |
| 1,44 | ppm 2H $C^{19}$-H |
| 1,42 | ppm 6H $(OCH_2-H_3)_2$ |
| 1,25/1,65 | ppm 2H $C^{19'}$-H |
| 1,15/1,11 | ppm 6H $CH(\underline{CH_3})_2$ |
| 0,98 | ppm 3H $C^{18'}$-H |
| 0,91 | ppm 3H $C^{18}$-H |

**EXEMPLE 6**

**(+) N-(désacétyl-0-4 vincristinoyl-23) amino-1 méthyl-2 propyl phosphonate de diéthyle**

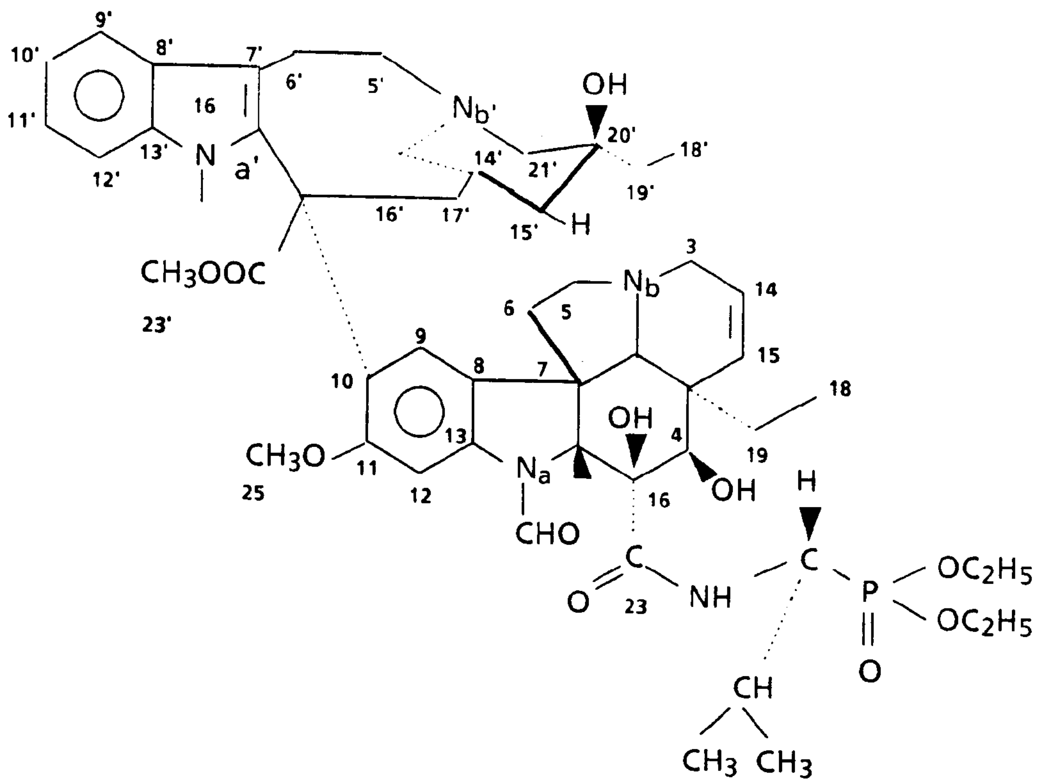

3,7 g du composé de l'exemple 4 sont dissous dans un mélange de 22 ml d'acide formique et 4 ml d'anhydride acétique. On maintient le milieu une heure à température ambiante et ensuite on le traite par 120 ml d'eau glacée. Le pH de la solution est alors amené à 9 par addition d'une solution glacée d'ammoniaque. Le milieu est extrait deux fois au dichlorométhane. La phase organique est lavée avec de l'eau salée, puis une fois à l'eau et séchée sur sulfate de magnésium anhydre. Le milieu est concentré sous vide. On obtient 3,5 g du produit attendu qu'on purifie par H.P.LC. préparative (500 g de Lichroprep RP 18 ; éluant $Na_2HPO_4$ $10^{-2}$M 40V:Méthanol 60V.) Les fractions 75 à 140 sont récupérées, le solvant est concentré sous vide, la phase aqueuse résiduelle est extraite au dichlorométhane. La phase organique est lavée à l'eau, séchée et concentrée sous vide. On obtient 500 mg de produit pur.

Rendement : 13 %

Spectre de résonance magnétique nucléaire du proton (solvant $CDCl_3$):

| | | | |
|---|---|---|---|
| 9,5 | ppm | 1H | CHO |
| 8,5 | ppm | 1H | NH |
| 8,1 | ppm | 3H | NH,NHCO, $C^9$-H |
| 7,65 | ppm | 1H | $C^{12}$-H |
| 7,50 | ppm | 1H | $C^{9'}$-H |
| 7,40 | ppm | | |
| 7,12 | ppm | 3H | $C^{10'}$-H+$C^{11'}$-H+$C^{12'}$-H |
| 7,02 | ppm | | |
| 5,85 | ppm | 1H | $C^{14}$-H |
| 5,70 | ppm | 1H | $C^{15}$-H |
| 4,42 | ppm | 1H | $C^{26}$-H |
| 4,36 | ppm | 2H | $C^{21'}$ |
| 4,10 | ppm | 5H | $C^4$-H+($O\underline{CH_2}CH_3)_2$ |
| 4,02 | ppm | 2H | $C^{21}$ |
| 3,91 | ppm | 1H | $C^2$-H |
| 3,75 | ppm | 3H | $C^{25}$-H |
| 3,65 | ppm | 3H | $C^{23'}$-H |
| 3,5/4,0 | ppm | 2H | $C^5$-H |
| 3,31 | ppm | 2H | $C^{5'}$-H |
| 2,45/3,10 | ppm | 2H | $C^3$-H |
| 2,40/3,21 | ppm | 2H | $C^{3'}$-H |
| 2,35/4,03 | ppm | 2H | $C^{17'}$-H |
| 2,14 | ppm | 1H | $\underline{CH}(CH_3)_2$ |
| 1,90/2,50 | ppm | 2H | $C^6$-H |
| 1,51 | ppm | 2H | $C^{15'}$-H |
| 1,40 | ppm | 2H | $C^{19}$-H |
| 1,38 | ppm | 6H | $(OCH_2-\underline{CH_3})_2$ |
| 1,25/1,60 | ppm | 2H | $C^{19'}$-H |
| 1,14 | ppm | 6H | $CH(\underline{CH_3})_2$ |
| 0,90 | ppm | 6H | $C^{18'}$-H |
| 0,72 | ppm | 3H | $C^{18}$-H |

**EXEMPLE 7**

**(-) N-(désacetyl-0-4 vincristinoyl-23) amino-1 methyl-2 propyl phosphonate de diéthyle**

Ce composé est obtenu selon le procédé dérit dans l'exemple 6 mais en utilisant le composé de l'exemple 5.

Le composé obtenu a été purifié par chromatographie, selon la méthode décrite dans l'exemple 6, mais en récupérant les fractions 200 à 290.

Spectre de résonance magnétique nucléaire du proton (solvant $CDCl_3$):

| | | |
|---|---|---|
| 8,12 | ppm 1H | $C^{12}$-H |
| 7,68 | ppm 1H | $C^{9}$-H |
| 7,53 | ppm 1H | $C^{9'}$-H |
| 7,44 | ppm 1H | $C^{12'}$-H |
| 7,12 | ppm 1H | $C^{10'}$-H |
| 7,02 | ppm 1H | $C^{11'}$-H |
| 5,91 | ppm 2H | $C^{14}$-H + $C^{15}$-H |
| 4,45 | ppm 1H | $C^{2}$-H |
| 4,20 | ppm 3H | $C^{26}$-H + $C^{21'}$-H |
| 4,10 | ppm 5H | $C^{21}$-H+$(O\underline{CH_2}-CH_3)_2$ |
| 3,85 | ppm 1H | $C^{17}$-H |
| 3,80 | ppm 3H | $C^{25}$-H |
| 3,63 | ppm 3H | $C^{23'}$-H |
| 3,6/3,9 | ppm 2H | $C^{5'}$-H |
| 3,55/4,1 | ppm 2H | $C^{5}$-H |
| 3,3/4,44 | ppm 2H | $C^{6'}$-H |
| 3,25/3,9 | ppm 2H | $C^{3}$-H |
| 2,83/3,8 | ppm 2H | $C^{3'}$-H |
| 2,27/3,9 | ppm 2H | $C^{17'}$-H |
| 2,12 | ppm 1H | $\underline{CH}(CH_3)_2$ |
| 1,78/2,51 | ppm 2H | $C^{6}$-H |
| 1,47 | ppm 2H | $C^{15'}$-H |
| 1,44 | ppm 2H | $C^{19'}$-H |
| 1,40/1,70 | ppm 2H | $C^{19}$-H |
| 1,31 | ppm 6H | $CH(\underline{CH_3})_2$ |
| 1,10 | ppm 1H | $C^{14'}$-H |
| 0,95/1,02 | ppm 6H | $(OCH_2\underline{CH_3})_2$ |
| 0,86 | ppm 3H | $C^{18}$-H |
| 0,70 | ppm 3H | $C^{18'}$-H |

Spectre de résonance magnétique nucléaire du $^{13}C$ (solvant $CDCl_3$):

| | | | |
|---|---|---|---|
| 173,3 | ppm $C^{23'}$ | 56,1 | ppm $C^{5'}$ |
| 169,5 | ppm $C^{23}$ | 55,1 | ppm $C^{16'}$ |
| 161,5 | ppm $C^{24}$ | 52,3 | ppm $C^{23'}$ |
| 156,7 | ppm $C^{11}$ | 50,4 | ppm $C^{7}$ |
| 141,2 | ppm $C^{13}$ | 49,4 | ppm $C^{3}$ |
| 136,0 | ppm $C^{13'}$ | 48,9/50,4 | ppm $C^{26}$ |
| 132,5 | ppm $C^{15}$ | 48,8 | ppm $C^{5}$ |
| 130,4 | ppm $C^{2'}$ | 44,1 | ppm $C^{3'}$ |
| 128,3 | ppm $C^{8'}$ | 41,8 | ppm $C^{20}$ |
| 126,4 | ppm $C^{10}$ | 38,8 | ppm $C^{6}$ |
| 123,7 | ppm $C^{8}$ + $C^{9}$ | 35,9 | ppm $C^{15'}$ |
| 121,7 | ppm $C^{14}$ + $C^{10'}$ | 35,1 | ppm $C^{17'}$ |
| 118,6 | ppm $C^{11'}$ | 33,9 | ppm $C^{19'}$ |
| 117,7 | ppm $C^{9'}$ | 31,4 | ppm $C^{19}$ |
| 113,0 | ppm $C^{7'}$ | 29,4 | ppm $\underline{C}H(CH_3)_2$ |
| 112,2 | ppm $C^{12'}$ | 26,2 | ppm $C^{14'}$ |
| 100,7 | ppm $C^{12}$ | 20,4 | ppm $C^{6'}$ |
| 82,3 | ppm $C^{16}$ | 17,4/20,1 | ppm $(OCH_2\underline{CH_3})_2$ |
| 72,3 | ppm $C^{17}$ | 16,2 | ppm $CH(\underline{CH_3})_2$ |
| 69,4 | ppm $C^{2}$ | 7,23 | ppm $C^{18'}$ |
| 66,5 | ppm $C^{20'}$ | 6,15 | ppm $C^{18}$ |
| 64,3 | ppm $C^{21'}$ | | |
| 61,6/62 | ppm $(O\underline{CH_2}-CH_3)_2$ | | |
| 60,3 | ppm $C^{21'}$ | | |
| 56,2 | ppm $C^{25}$ | | |

**EXEMPLE 8**

**(+) N-($N_a$-desformyl désacétyl-O-4 vincristinoyl-23) amino-1 éthyl phosphonate de diéthyle**

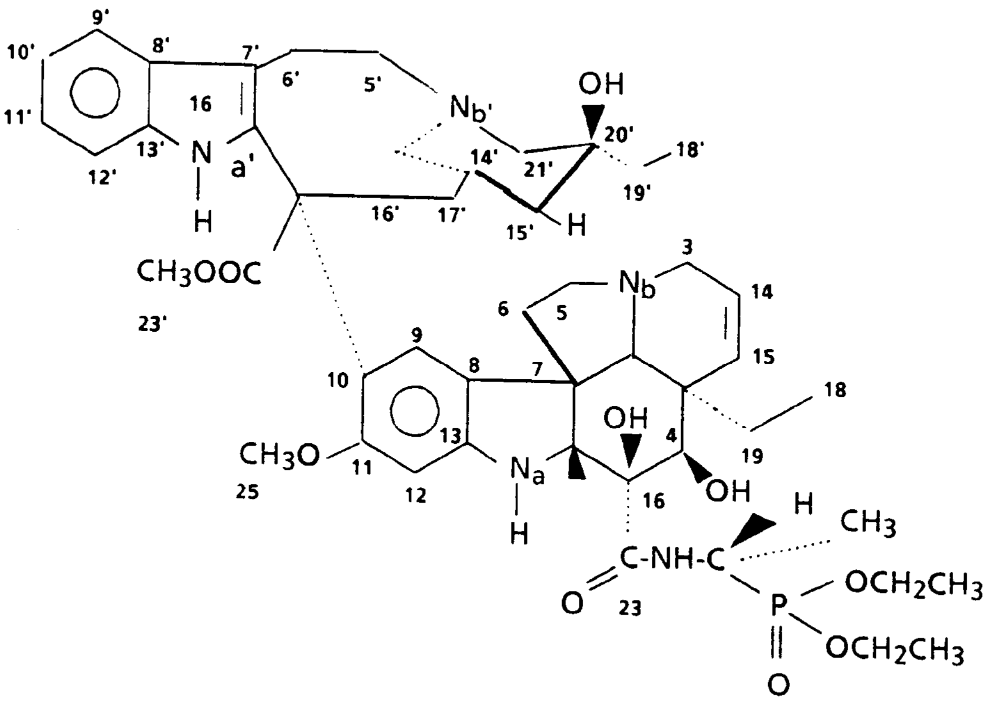

Ce composé a été préparé selon le procédé décrit dans l'exemple 4 mais en utilisant le (+) amino-1-éthyl phosphonate de diéthyle.

Spectre de résonance magnétique nucléaire du proton (solvant $CDCl_3$):

| | |
|---|---|
| 7,62 ppm 1H $C^{9'}$-H | 2,90/3,35 ppm 2H $C^{3}$-H |
| 7,35 | 2,80 ppm 2H $C^{21'}$-H |
| 7,15 ppm 3H $C^{10'}$-H+$C^{11'}$-H+$C^{12'}$-H | 2,60 ppm 2H $C^{21}$-H |
| 7,08 | 2,20/3,10 ppm 2H $C^{3'}$-H |
| 6,60 ppm 1H $C^{9}$-H | 2,40/3,20 ppm 2H $C^{5}$-H |
| 6,10 ppm 1H $C^{12}$-H | 2,50/4,00 ppm 2H $C^{17'}$-H |
| 5,80 ppm 1H $C^{14}$-H | 1,60/2,00 ppm 2H $C^{6}$-H |
| 5,60 ppm 1H $C^{15}$-H | 1,60 ppm 2H $C^{15'}$-H |
| 4,50 ppm 1H $C^{26}$-H | 1,50 ppm 3H $\underline{CH_3}$-$CH_2$ |
| 4,18 ppm 1H $C^{4}$-H | 1,45 ppm 2H $C^{19}$-H |
| 4,11 ppm 4H $(O\underline{CH_2}CH_3)_2$ | 1,30 ppm 6H $(OCH_2\underline{CH_3})_2$ |
| 3,85 ppm 1H $C^{2}$-H | 1,30/1,70 ppm 2H $C^{19'}$-H |
| 3,80 ppm 3H $C^{25}$-H | 0,95 ppm 3H $C^{18'}$-H |
| 3,60 ppm 3H $C^{23'}$-H | 0,80 ppm 3H $C^{18}$-H |
| 3,35 ppm 2H $C^{5'}$-H | |

**EXEMPLE 9**

**(+) N-(désacétyl-0-4 vincristinoyl-23) amino-1 éthyl phosphonate de diéthyle**

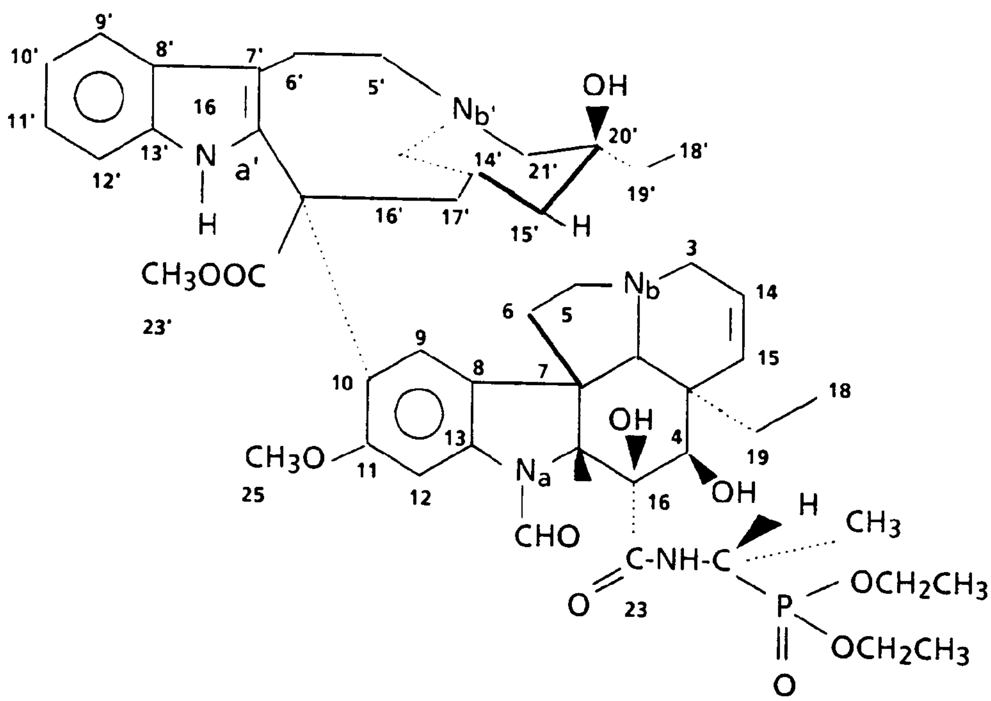

**Procédé A**

Ce composé a été préparé à partir du composé de l'exemple 8, en utilisant le procédé décrit dans l'exemple 6.

<u>Spectre de résonance magnétique nucléaire du proton (solvant $CDCl_3$)</u>:

| | | | |
|---|---|---|---|
| 8,00 | ppm | 1H | $C^{12}$-H |
| 7,70 | ppm | 1H | $C^{9}$-H |
| 7,55 | ppm | 1H | $C^{9'}$-H |
| 7,50 | ppm | 1H | $C^{12'}$-H |
| 7,10 | ppm | 2H | $C^{10'}$-H + $C^{11'}$-H |
| 5,95 | ppm | 1H | $C^{14}$-H |
| 5,90 | ppm | 1H | $C^{15}$-H |
| 4,30 | ppm | 1H | $C^{2}$-H |
| 4,25 | ppm | 1H | $C^{26}$-H |
| 4,20 | ppm | 3H | $C^{21}$-H + $C^{21'}$-H |
| 4,05 | ppm | 4H | $(O-\underline{CH_2}-CH_3)_2$ |
| 3,70 | ppm | 3H | $C^{25}$-H |
| 3,65 | ppm | 3H | $C^{23'}$-H |
| 3,40/3,90 | ppm | 2H | $C^{5}$-H |
| 3,40/4,10 | ppm | 2H | $C^{6'}$-H |
| 3,30/3,80 | ppm | 2H | $C^{5'}$-H |
| 3,30/3,75 | ppm | 2H | $C^{3}$-H |
| 2,80/3,70 | ppm | 2H | $C^{3'}$-H |
| 2,30/3,90 | ppm | 2H | $C^{17'}$-H |
| 1,70/2,40 | ppm | 2H | $C^{6}$-H |
| 1,50 | ppm | 2H | $C^{15'}$-H |
| 1,40 | ppm | 3H | $\underline{CH_3}-CH$ |
| 1,40/1,70 | ppm | 2H | $C^{19}$-H |
| 1,30/1,60 | ppm | 2H | $C^{19'}$-H |
| 1,05 | ppm | 6H | $(OCH_2-\underline{CH_3})$ |
| 1,00 | ppm | 1H | $C^{14'}$-H |
| 0,95 | ppm | 3H | $C^{18}$-H |
| 0,90 | ppm | 3H | $C^{18'}$-H |

**Procédé B**

Une solution de 300 mg du sulfate de (+) N-(désacétyl-O-4 vinblastinoyl-23) amino-1 éthyl phosphonate de diéthyle (demande de brevet EP 318 392) dans 28 ml de chlorure de méthylène et 4 ml d'acide acétique est refroidie à 70°C après avoir été dégazée par barbotage d'argon à température ambiante.

On additionne goutte à goutte une solution de 103 mg de permanganate de potassium et 290 mg d'éther couronne 18-6 dans 12 ml de chlorure de méthylène.

La réaction terminée, le milieu réactionnel est versé sur une solution glacée de 19 ml de disulfite de sodium à 5 % et de 10 ml d'ammoniaque concentrée.

L'émulsion résultante est filtrée sur célite et le filtrat est extrait à l'aide de 3 fois 25 ml de chlorure de méthylène.

Les phases organiques réunies sont lavées à l'aide de 10 ml d'eau et séchées sur sulfate de magnésium, l'évaporation du solvant fournit la base attendue.

Pour obtenir le sulfate correspondant, on ajoute une quantité adéquate d'éthanol sulfurique.

Rendement global : 20 %

**ETUDE PHARMACOLOGIQUE**

**EXEMPLE 10**

**<u>Activité antitumorale sur la leucémie P 388 chez la souris</u>**

Des souris (n=6), de souche $B_6D_2F_1$ ($F_1$:C57$Bl_6$ X $DBA_2$), ont reçu, par voie intrapéritonéale, au jour zéro, 0,4ml de sérum physiologique contenant en suspension $10^6$ de cellules leucémiques. Les produits à examiner

ont été administrés aux groupes "essais", par voie i.p., un jour après l'inoculation de la leucémie. La mortalité des animaux des groupes "essais", et "contrôles" a été enregistrée pendant 60 jours après l'inoculation. Dans le Tableau I est indiqué le nombre des survivants consignés à l'issue de 30 jours et 60 jours d'observation. Après 60 jours d'observation, les animaux ayant survécus ont été considérés en rémission à long terme. Le Tableau II indique les valeurs du pourcentage de la durée de vie moyenne (T.M.S.) des groupes essais T sur la vie moyenne du groupe contrôle C non traité. Des valeurs de T/C (TMS) supérieures à 125% sont significatives d'une activité antitumorale.

Comme les résultats des Tableaux I et II le démontrent, les composés de l'invention possèdent une meilleure activité antitumorale par rapport aux composés de référence vinblastine et vincristine, ainsi que par rapport au (+) N-(désacétyl-O-4 vinblastinoyl-23) amino-1 méthyl-2 propyl phosphonate de diéthyle (N.D.V.A.M.P.P.) qui est le composé le plus actif parmi les produits décrits dans la demande de brevet EP 318392. En effet, le composé de l'exemple 6 pour une dose deux fois inférieure à celle du N.D.V.A.M.P.P. est curative pour la totalité des souris.

TABLEAU I

| COMPOSES | DOSE mg/kg | SURVIVANTS | |
|---|---|---|---|
| | | 30 jours | 60 jours |
| VINBLASTINE | 3 mg/kg | 1/6 | 1/6 |
| VINCRISTINE | 3 mg/kg | 0/6 | 0/6 |
| N.D.V.A.M.P.P. | 0,2 mg/kg | 5/6 | 5/6 |
| EXEMPLE 6 | 0,1 mg/kg | 6/6 | 6/6 |

TABLEAU II

| COMPOSES | DOSE mg/kg | T/C % |
|---|---|---|
| VINBLASTINE | 3 mg/kg | 245 |
| VINCRISTINE | 3 mg/kg | 192 |
| N.D.V.A.M.P.P. | 0,2 mg/kg | 405 |
| EXEMPLE 6 | 0,1 mg/kg | ) 550 |

**EXEMPLE 11**

**Activité antitumorale sur un cancer du poumon humain greffé chez la souris NUDE (LX1)**

Les souris NUDE sont traitées quand la taille de la tumeur est de 5 mm x 5 mm. Les composés sont administrés à J0, J3, J6 et J9 par voie i.p.. Comme les résultats du Tableau III le démontrent, les composés de l'invention possèdent une activité antitumorale très importante, alors que la vinblastine est très peu active.

TABLEAU III

| COMPOSES | DOSE mg/kg | SGD * | T/C % ** |
|---|---|---|---|
| VINBLASTINE | 1 | 1,5 (+) | 56 (+) |
| EXEMPLE 2 | 0,70 | 3,1 (+++) | 21 (+++) |

* : SGD : Standard growth delay

** : Volume tumeur animaux traités/volume tumeur animaux témoins

**EXEMPLE 12**

Cytotoxicité in vitro

Les cellules L1210, en phase exponentielle de croissance, sont diluées par du milieu de culture complet (RPMI, contenant 10 % de serum de veau foetal, 2 nM de glutamine, 50 Unités/ ml de penicilline, 50 µg/ml de streptomycine, 7 10 nM Hepes) de façon à obtenir une densité de $10^4$ cellules/ml.

Les produits sont testés à 9 concentrations (dilutions séries de 2 en 2) et incubés avec les cellules pendant 48 heures. Le nombre de cellules viables est ensuite quantifié par un essai colorimétrique, le Microculture Tetrazolium Assay.

Les résultats sont exprimés en $IC_{50}$, concentration en produit qui inhibe à 50 % la prolifération des cellules témoins.

Comme les résultats du tableau IV le démontrent, les composés de l'invention possèdent une meilleure activité antitumorale que les composés de référence vinblastine et vincristine.

TABLEAU IV

| COMPOSES | $IC_{50}$ |
|---|---|
| Vincristine | 4,20 ± 0,7 nM |
| Vinblastine | 2,30 ± 0,5 nM |
| Exemple 2 | 0,30 ± 0,09 nM |
| Exemple 6 | 0,35 ± 0,09 nM |

**PREPARATION PHARMACEUTIQUE**

**EXEMPLE 13**

**Poudre lyophilisée pour préparation injectable contenant 0,2 mg de (+) N-(désacétyl)-0-4 vincristinoyl-23) amino-1 méthyl-2 propyl phosphonate de diéthyle**

(+) N-(désacétyl-0-4 vincristinoyl-23) amino-1 méthyl-2 propyl phosphonate de diéthyle . . . . . . . . . . . . . . . . . . . . . 0,2 mg

Lactose anhydre . . . . . . . . . . . . . . . . . . . . 10,0 mg

pour un flacon de poudre.

**Revendications**

**Revendications pour les Etats contractants suivants : AT, BE, CH, DE, DK, FR, GB, IT, LI, LU, NL, SE**

1. Composés de formule I :

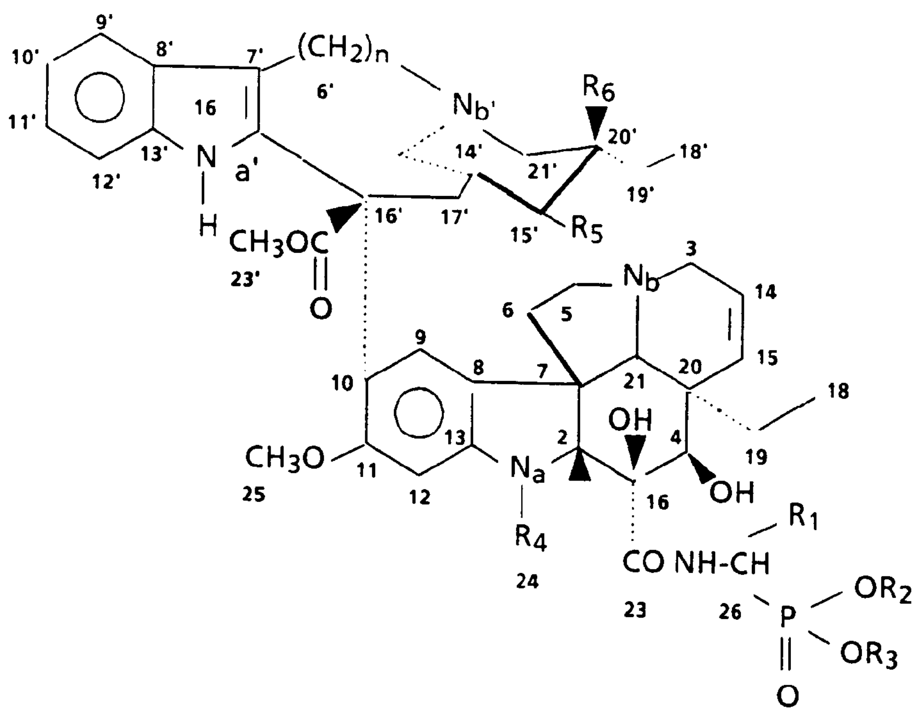

(I)

dans laquelle:

- $R_1$ représente un atome d'hydrogène, un radical alkyle linéaire ou ramifié renfermant de 1 à 6 atomes de carbone, un radical alcényle linéaire ou ramifié renfermant de 2 à 6 atomes de carbone, un radical arylalkyle de 7 à 10 atomes de carbone pouvant porter comme substituant sur le cycle aromatique un atome d'halogène, un radical hydroxyle ou un radical alkyle ou alcoxy renfermant chacun de 1 à 5 atomes de carbone, un radical indolyl-2 méthyle, un radical imidazolyl-4 méthyle, ou un radical alcoxycarbonylméthyle renfermant de 3 à 11 atomes de carbone,
- $R_2$ et $R_3$ identiques ou différents représentent chacun un radical alkyle linéaire ou ramifié renfermant

de 1 à 6 atomes de carbone,

- n est égal à 1 ou 2,
- $R_4$ représente un atome d'hydrogène, un radical formyle ou un radical méthyle, à condition toutefois que $R_4$ ne soit jamais un radical méthyle quand n est égal à 2, et
- $R_5$ et $R_6$ soit forment ensemble une double liaison, soit $R_6$ représente un atome d'hydrogène et $R_6$ un radical hydroxy,

sous forme de mélange de diastéréo-isomères ou d'isomères purs, leurs $N_{b'}$-oxydes et leurs sels d'addition à un acide minéral ou organique pharmaceutiquement acceptable.

**2.** Le N-(désacétyl-0-4 noranhydro-5′ vinblastinoyl-23) amino-1 méthyl-2 propyl phosphonate de diéthyle, composé répondant à la formule I selon la revendication 1, sous forme de mélange de diastéréo-isomères ou d'isomères purs, et ses sels d'addition à un acide minéral ou organique pharmaceutiquement acceptable.

**3.** Le (+) N-($N_a$-desformyl désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable.

**4.** Le (+) N-(désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable.

**5.** Le (-) N-(désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable

**6.** Le (+) N-(désacétyl-0-4 vincristinoyl-23) amino-1 éthyl phosphonate de diéthyle, composé répondant à la formule I, selon la revendication 1, et ses sels d'addition à un acide minéral ou organique pharmaceutiquement acceptable

**7.** Procédé de préparation des composés de formule générale I, caractérisé,
soit
en ce que l'on fait réagir une amine, sous forme racémique ou optiquement pure, de formule générale II :

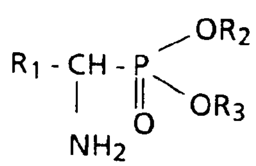

(II)

dans laquelle la définition de $R_1$, $R_2$ et $R_3$ demeure celle définie précédemment pour la formule générale I, selon la revendication 1,
avec un composé de formule III :

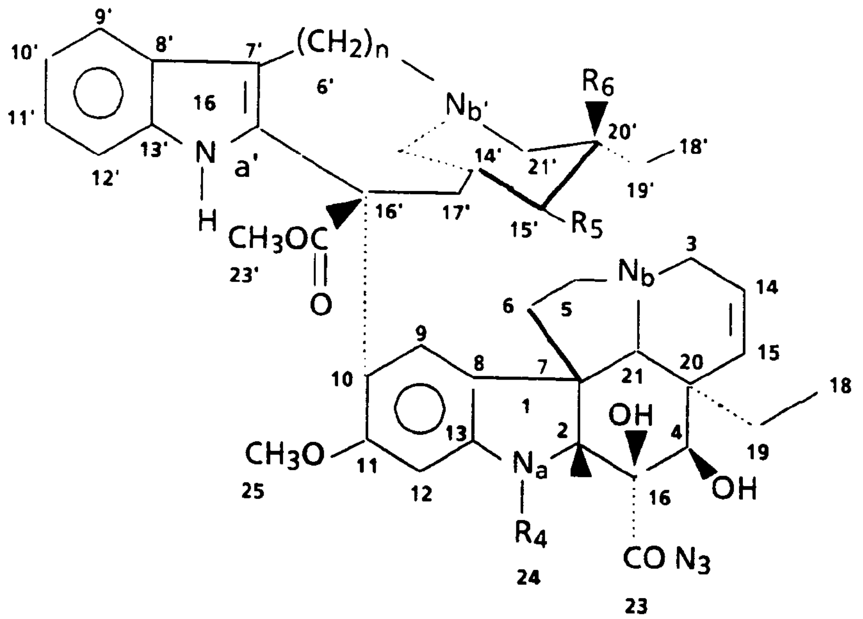

(III)

dans laquelle n, $R_5$ et $R_6$ ont la signification définie précédemment pour la formule générale I, selon la revendication 1, et $R_4$ représente un atome d'hydrogène ou un radical méthyle, pour former respectivement sous forme de mélange de diastéréo-isomères ou d'isomères purs les composés de formule générale I, selon la revendication 1, dans laquelle n, $R_1$, $R_2$, $R_3$, $R_5$ et $R_5$ ont la signification définie précédemment et $R_4$ représente un atome d'hydrogène ou un radical méthyle,
puis pour former les composés de formule générale I, selon la revendication 1, dans laquelle $R_4$ représente un radical formyle,
qu'on soumet les composés de formule Ia :

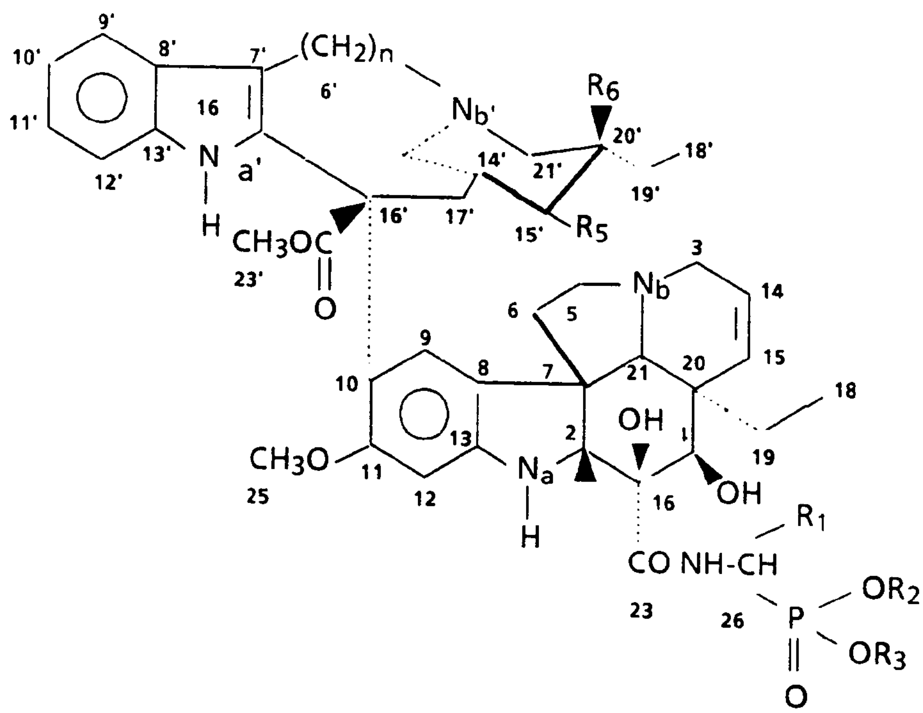

(Ia)

à l'action d'acide formique en présence d'anhydride acétique,
soit
en ce que l'on traite un composé sous forme racémique ou optiquement actif pur de formule IV :

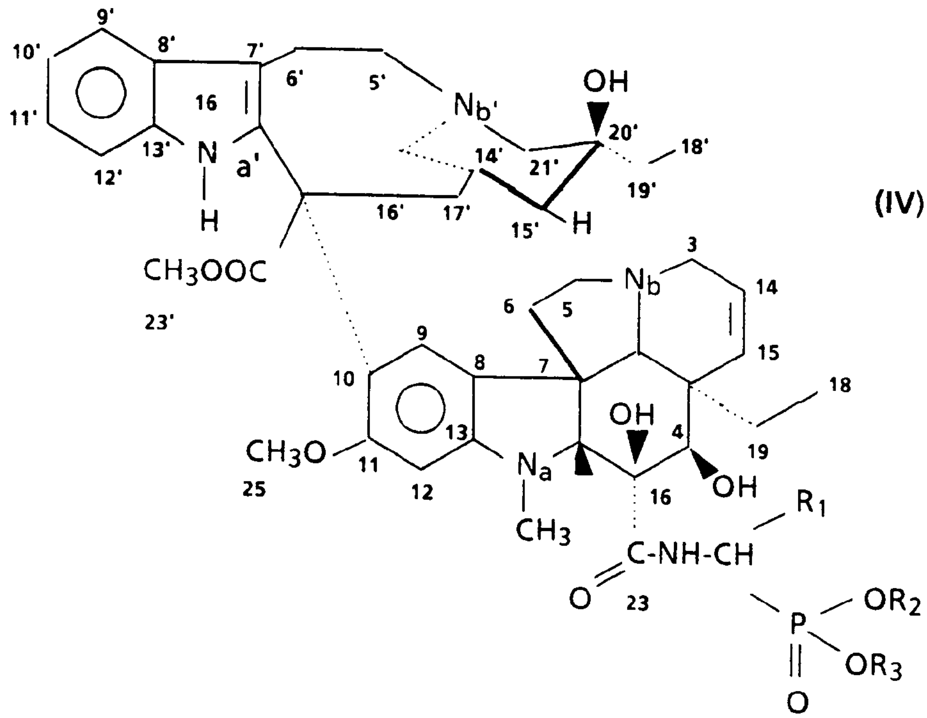

(IV)

dans laquelle la définition de $R_1$, $R_2$ et $R_3$ demeure identique à celle définie précédemment pour la formule I, selon la revendication 1,

par l'ion permanganate en milieu acide dans un solvant inerte, à une température comprise entre -40°C et -75°C,

pour former les composés de formule générale I, selon la revendication 1, dans laquelle n est égal à 2 et $R_4$ représente un radical formyle,

et ensuite,

que l'on salifie les composés de formule générale I, selon la revendication 1, avec un acide minéral ou organique pharmaceutiquement acceptable,

ou

que l'on transforme aux $N_{b'}$-oxydes correspondants à l'aide d'un solvant organique basique saturé d'oxygène.

8. Composition pharmaceutique contenant comme principe actif un composé selon l'une quelconque des revendications 1 à 6, en association ou en mélange avec un excipient ou un véhicule inerte, non toxique, phamaceutiquement acceptable.

9. Composition pharmaceutique selon la revendication 8 refermant le principe actif à la dose de 0,1 à 100 mg.

10. Composition pharmaceutique selon les revendications 8 et 9 renfermant comme principe actif au moins un composé selon les revendications 1 à 4 utilisable pour le traitement des maladies néoplastiques chez l'être vivant.

**Revendications pour l'Etat contractant suivant : ES**

1. Procédé de préparation des composés de formule I :

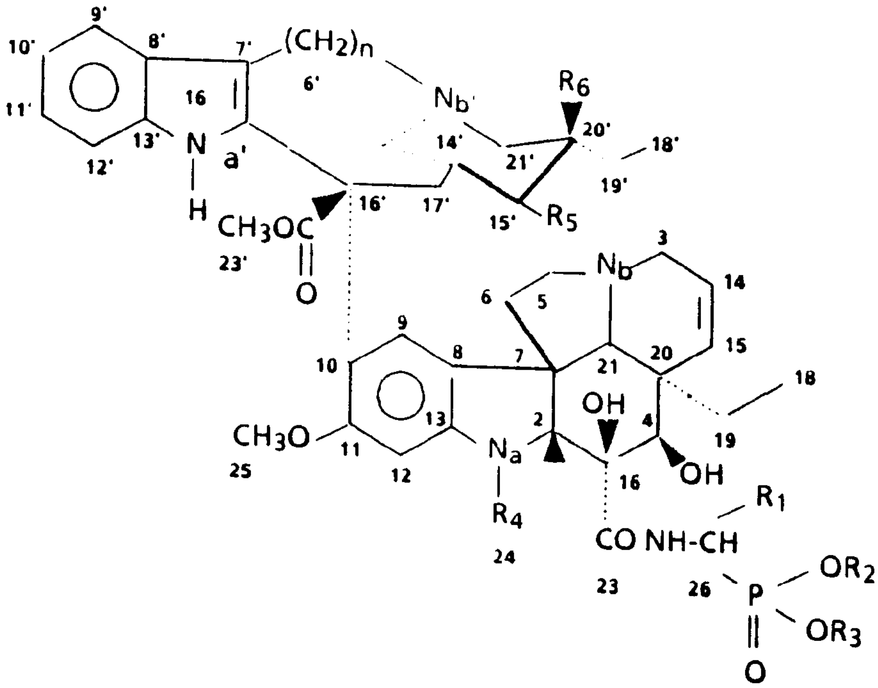

(I)

dans laquelle:

- $R_1$ représente un atome d'hydrogène, un radical alkyle linéaire ou ramifié renfermant de 1 à 6 atomes de carbone, un radical alcényle linéaire ou ramifié renfermant de 2 à 6 atomes de carbone, un radical arylalkyle de 7 à 10 atomes de carbone pouvant porter code substituant sur le cycle aromatique un atome d'halogène, un radical hydroxyle ou un radical alkyle ou alcoxy renfermant chacun de 1 à 5 atomes de carbone, un radical indolyl-2 méthyle, un radical imidazolyl-4 méthyle, ou un radical alcoxycarbonylméthyle renfermant de 3 à 11 atomes de carbone,
- $R_2$ et $R_3$ identiques ou différents représentent chacun un radical alkyle linéaire ou ramifié renfermant de 1 à 6 atomes de carbone,
- n est égal à 1 ou 2,
- $R_4$ représente un atome d'hydrogène, un radical formyle ou un radical méthyle, à condition toutefois que $R_4$ ne soit jamais un radical méthyle quand n est égal à 2, et
- $R_5$ et $R_6$ soit forment ensemble une double liaison, soit $R_6$ représente un atome d'hydrogène et $R_6$ un radical hydroxy,

sous forme de mélange de diastéréoisomères ou d'isomères purs, leurs $N_{b'}$-oxydes et leurs sels d'addition à un acide minéral ou organique pharmaceutiquement acceptable,
caractérisé,
<u>soit</u>

en ce que l'on fait réagir une amine, sous forme racémique ou optiquement pure, de formule générale II :

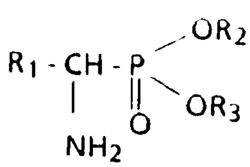

(II)

dans laquelle la définition de $R_1$, $R_2$ et $R_3$ demeure celle définie précédemment pour la formule générale I,
avec un composé de formule III :

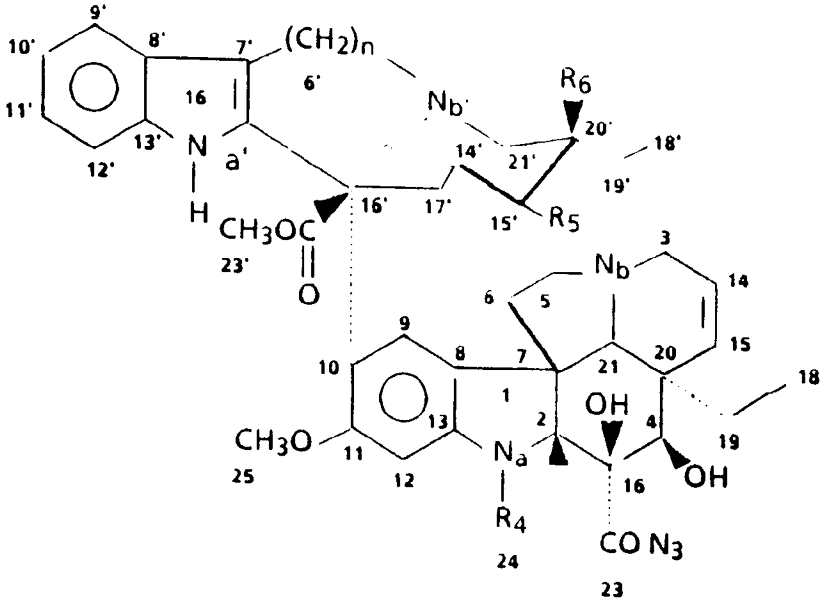

(III)

dans laquelle n, $R_5$ et $R_6$ ont la signification définie précédemment pour la formule générale I, et $R_4$ représente un atome d'hydrogène ou un radical méthyle, pour former respectivement sous forme de mélange de diastéréoisomères ou d'isomères purs les composés de formule générale I, dans laquelle n, $R_1$, $R_2$, $R_3$, $R_5$ et $R_6$ ont la signification définie précédemment et $R_4$ représente un atome d'hydrogène ou un radical méthyle,
puis pour former les composés de formule générale I, dans laquelle $R_4$ représente un radical formyle, qu'on soumet les composés de formule Ia :

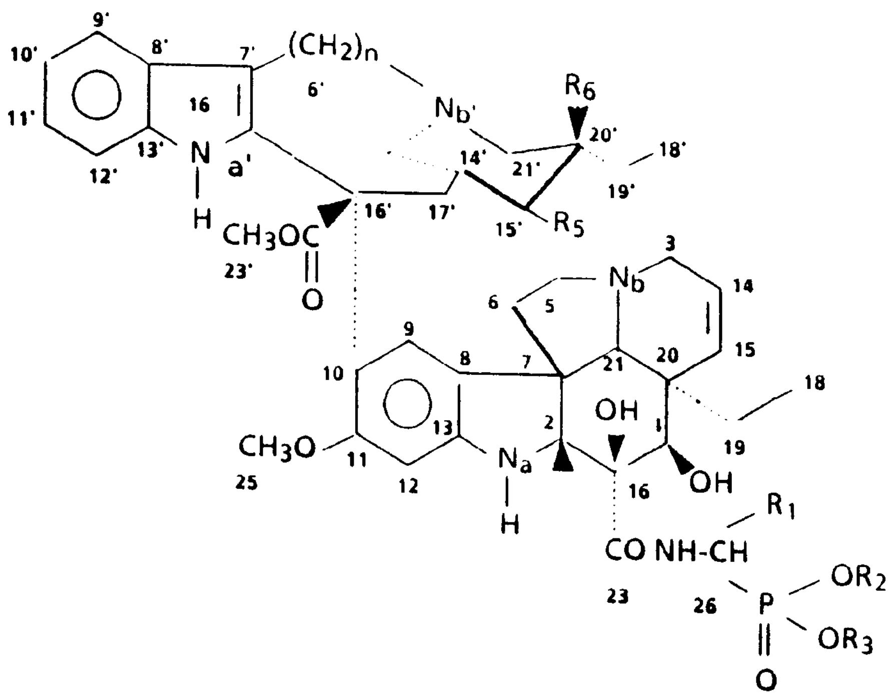

(Ia)

à l'action d'acide formique en présence d'anhydride acétique,
<u>soit</u>

en ce que l'on traite un composé sous forme racémique ou optiquement actif pur de formule IV :

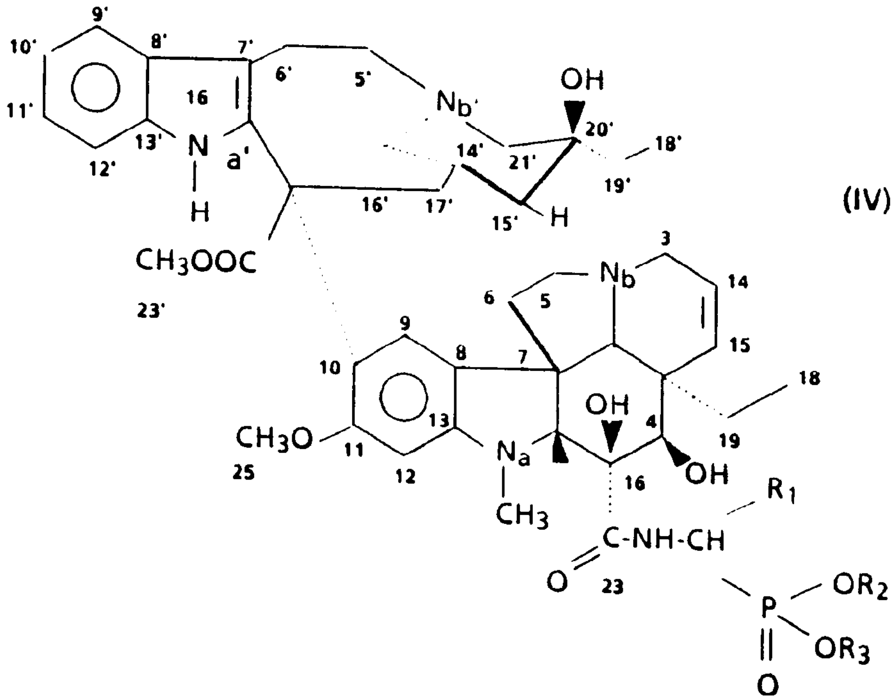

(IV)

dans laquelle la définition de $R_1$, $R_2$ et $R_3$ demeure identique à celle définie précédemment pour la formule I,

par l'ion permanganate en milieu acide dans un solvant inerte, à une température comprise entre -40°C et -75°C,

pour former les composés de formule générale I, dans laquelle n est égal à 2 et $R_4$ représente un radical formyle,

et ensuite,

que l'on salifie les composés de formule générale I, avec un acide minéral ou organique pharmaceutiquement acceptable,

ou

que l'on transforme aux $N_{b'}$-oxydes correspondants à l'aide d'un solvant organique basique saturé d'oxygène.

2. Le N-(désacétyl-0-4 noranhydro-5′ vinblastinoyl-23) amino-1 méthyl-2 propyl phosphonate de diéthyle, composé répondant à la formule I selon la revendication 1, sous forme de mélange de diastéréo-isomères ou d'isomères purs, et ses sels d'addition à un acide minéral ou organique pharmaceutiquement acceptable, lorsque préparé selon le procédé de la revendication 1 ou un autre procédé chimiquement équivalent.

3. Le (+) N-($N_a$-desformyl désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable, lorsque préparé selon le procédé de la revendication 1 ou un autre procédé chimiquement équivalent.

4. Le (+) N-(désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable, lorsque préparé selon le procédé de la revendication 1 ou un autre procédé chimiquement équivalent.

5. Le (-) N-(désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable, lorsque préparé selon le procédé de la revendication 1 ou un autre procédé chimiquement équivalent.

6. Le (+) N-(désacétyl-0-4 vincristinoyl-23) amino-1 éthyl phosphonate de diéthyle, composé répondant à

la formule I, selon la revendication 1, et ses sels d'addition à un acide minéral ou organique pharmaceutiquement acceptable, lorsque préparé selon le procédé de la revendication 1 ou un autre procédé chimiquement équivalent.

**Revendications pour l'Etat contractant suivant : GR**

1. Procédé de préparation des composés de formule I :

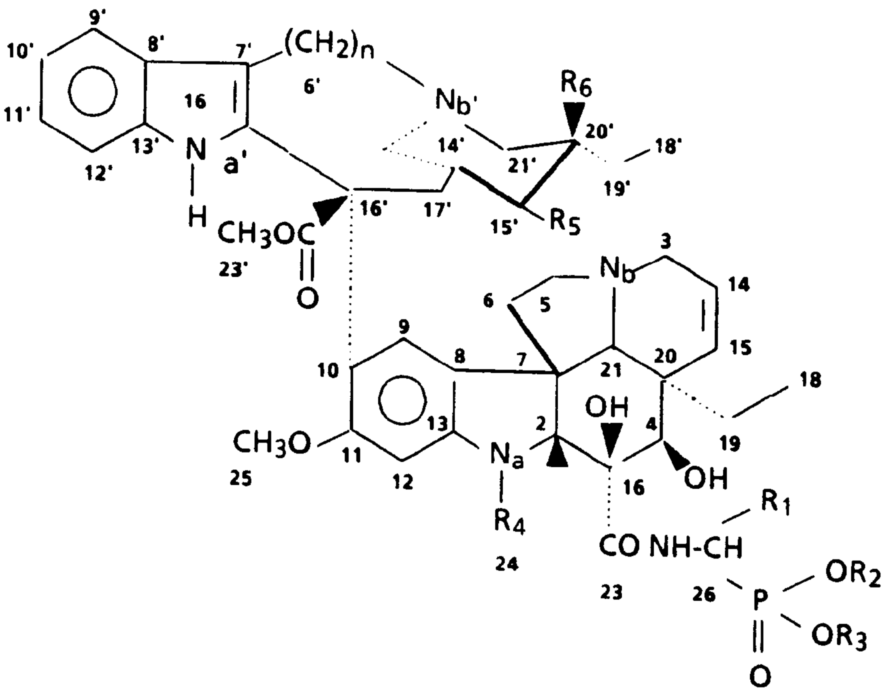

(I)

dans laquelle:

- $R_1$ représente un atome d'hydrogène, un radical alkyle linéaire ou ramifié renfermant de 1 à 6 atomes de carbone, un radical alcènyle linéaire ou ramifié renfermant de 2 à 6 atomes de carbone, un radical arylalkyle de 7 à 10 atomes de carbone pouvant porter comme substituant sur le cycle aromatique un atome d'halogène, un radical hydroxyle ou un radical alkyle ou alcoxy renfermant chacun de 1 à 5 atomes de carbone, un radical indolyl-2 méthyle, un radical imidazolyl-4 méthyle, ou un radical alcoxycarbonylméthyle renfermant de 3 à 11 atomes de carbone,
- $R_2$ et $R_3$ identiques ou différents représentent chacun un radical alkyle linéaire ou ramifié renfermant de 1 à 6 atome de carbone,
- n est égal à 1 ou 2,
- $R_4$ représente un atome d'hydrogène, un radical formyle ou un radical méthyle, à condition toutefois que $R_4$ ne soit jamais un radical méthyle quand n est égal à 2,
  et
- $R_5$ et $R_6$ soit forment ensemble une double liaison, soit $R_6$ représente un atome d'hydrogène et $R_6$ un radical hydroxy,

sous forme de mélange de diastéréo-isomères ou d'isomères purs, leurs $N_{b'}$-oxydes et leurs sels d'addition à un acide minéral ou organique pharmaceutiquement acceptable,
caractérisé,
<u>soit</u>

en ce que l'on fait réagir une amine, sous forme racémique ou optiquement pure, de formule générale II :

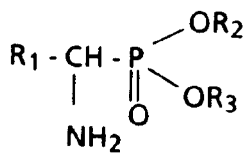

(II)

dans laquelle la définition de $R_1$, $R_2$ et $R_3$ demeure celle définie précédemment pour la formule générale I,
avec un composé de formule III :

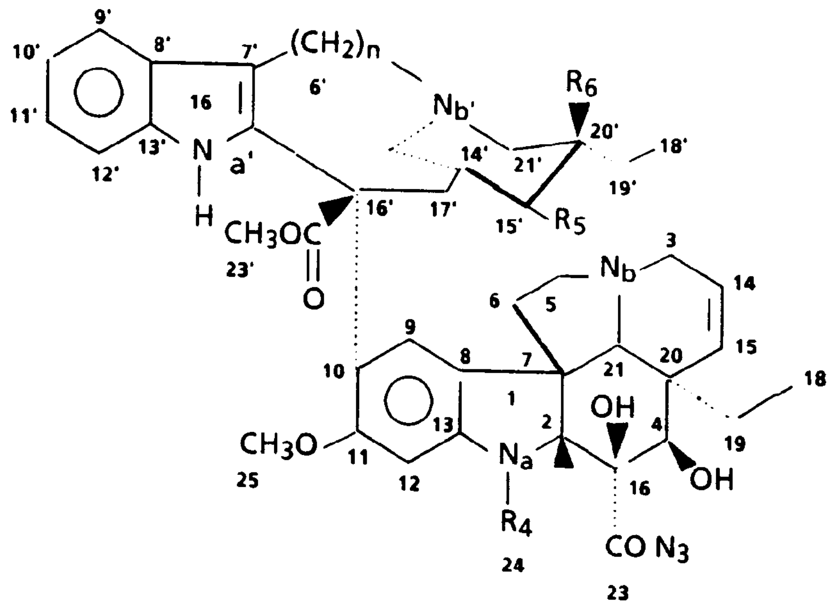

(III)

dans laquelle n, $R_5$ et $R_6$ ont la signification définie précédemment pour la formule générale I, et $R_4$ représente un atome d'hydrogène ou un radical méthyle, pour former respectivement sous forme de mélange de diastéréoisomères ou d'isomères purs les composés de formule générale I, dans laquelle n, $R_1$, $R_2$, $R_3$, $R_5$ et $R_6$ ont la signification définie précédemment et $R_4$ représente un atome d'hydrogène ou un radical méthyle,
puis pour former les composés de formule générale I, dans laquelle $R_4$ représente un radical formyle, qu'on soumet les composés de formule Ia :

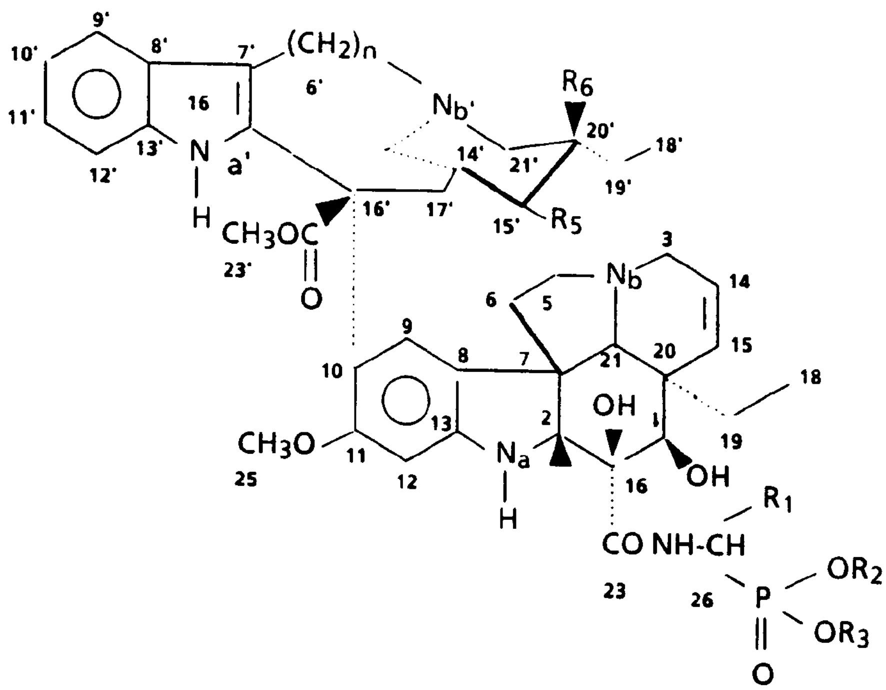

(Ia)

l'action d'acide formique en présence d'anhydride acétique,

soit

en ce que l'on traite un composé sous forme racémique ou optiquement formule actif pur de formule IV :

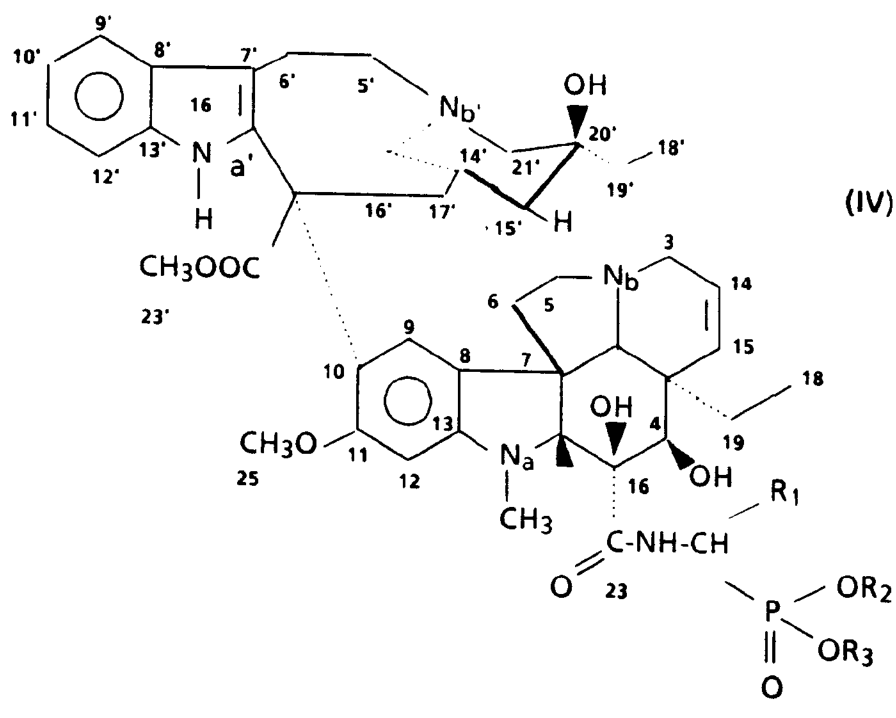

(IV)

dans laquelle la définition de $R_1$, $R_2$ et $R_3$ demeure identique à celle définie précédemment pour la formule I,

par l'ion permanganate en milieu acide dans un solvant inerte, à une température comprise entre -40°C et -75°C,

pour former les composés de formule générale I, dans laquelle n est égal à 2 et $R_4$ représente un radical formyle,

et ensuite,

que l'on salifie les composés de formule générale I, avec un acide minéral ou organique pharmaceutiquement acceptable,

ou

que l'on transforme aux $N_{b'}$-oxydes correspondants à l'aide d'un solvant organique basique saturé d'oxygène.

2. Le N-(désacétyl-0-4 noranhydro-5′ vinblastinoyl-23) amino-1 méthyl-2 propyl phosphonate de diéthyle, composé répondant à la formule I selon la revendication 1, sous forme de mélange de diastéréo-isomères ou d'isomères purs, et ses sels d'addition à un acide minéral ou organique pharmaceutiquement acceptable, lorsque préparé selon le procédé de la revendication 1 ou un autre procédé chimiquement équivalent.

3. Le (+) N-($N_a$-desformyl désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable, lorsque préparé selon le procédé de la revendication 1 ou un autre procédé chimiquement équivalent.

4. Le (+) N-(désacétyl-0-4 vincristinoyl-23) amino-1 méthyl-2 propyl phosphonate de diéthyle, composé répondant à la formule I selon la revendication 1, et ses sels d'addition à un acide minéral ou organique pharmaceutiquement acceptable, lorsque préparé selon le procédé de la revendication 1 ou un autre procédé chimiquement équivalent.

5. Le (-) N-(désacétyl-0-4 vincristinoyl-23) amino-1 méthyl-2 propyl phosphonate de diéthyle, composé répondant à la formule I, selon la revendication 1, et ses sels d'addition à un acide minéral ou organique pharmaceutiquement acceptable, lorsque préparé selon le procédé de la revendication 1 ou un autre procédé chimiquement équivalent.

6. Le (+) N-(désacétyl-0-4 vincristinoyl-23) amino-1 éthyl phosphonate de diéthyle, composé répondant à la formule I, selon la revendication 1, et ses sels d'addition à un acide minéral ou organique pharmaceutiquement acceptable, lorsque préparé selon le procédé de la revendication 1 ou un autre procédé chimiquement équivalent.

## Patentansprüche

## Patentansprüche für folgende Vertragsstaaten : AT, BE, CH, DE, DK, FR, GB, IT, LI, LU, NL, SE

1. Verbindungen der Formel I:

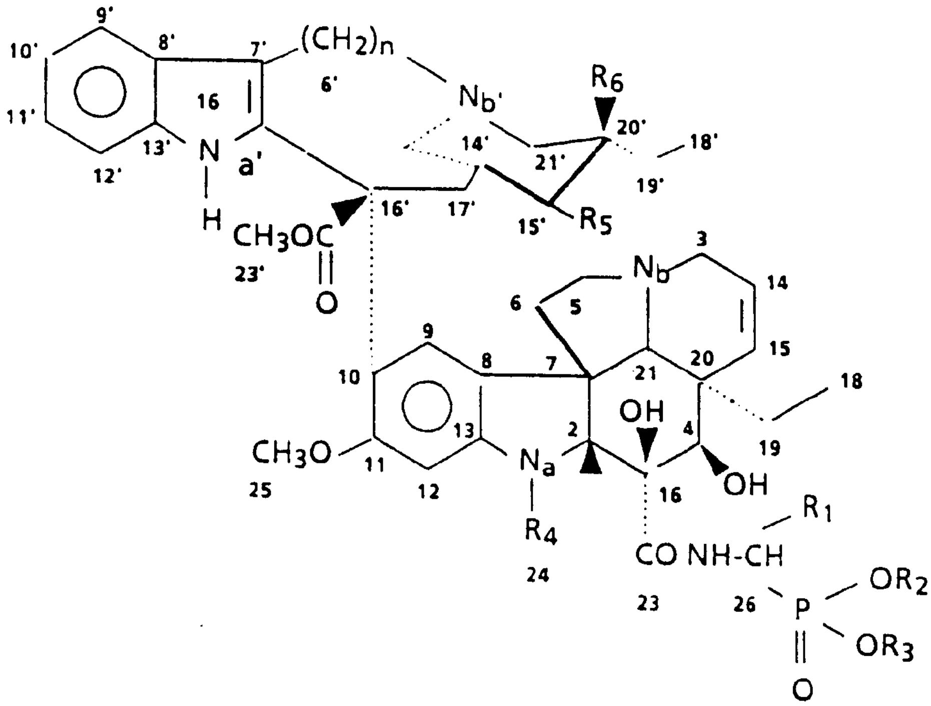

(I)

in der

- $R_1$ ein Wasserstoffatom, eine geradkettige oder verzweigte Alkylgruppe mit 1 bis 6 Kohlenstoffatomen, eine geradkettige oder verzweigte Alkenylgruppe mit 2 bis 6 Kohlenstoffatomen, eine Arylalkylgruppe mit 7 bis 10 Kohlenstoffatomen, die als Substituent am aromatischen Ring ein Halogenatom, eine Hydroxylgruppe oder eine Alkyl- oder Alkoxygruppe mit jeweils 1 bis 5 Kohlenstoffatomen tragen kann, eine Indol-2-yl-methylgruppe, eine Imidazol-4-yl-methylgruppe oder eine Alkoxycarbonylmethylgruppe mit 3 bis 11 Kohlenstoffatomen,
- $R_2$ und $R_3$, die gleichartig oder verschieden sein können, jeweils eine geradkettige oder verzweigte Alkylgruppe mit 1 bis 6 Kohlenstoffatomen,
- n 1 oder 2,
- $R_4$ ein Wasserstoffatom, eine Formylgruppe oder eine Methylgruppe mit der Maßgabe, daß $R_4$ keine Methylgruppe darstellt, wenn n den Wert 2 besitzt, und
- $R_5$ und $R_6$ entweder gemeinsam eine Doppelbindung oder $R_5$ ein Wasserstoffatom und $R_6$ eine Hydroxylgruppe

bedeuten,
in Form einer Mischung der Diastereoisomeren oder der reinen Isomeren, deren $N_{b'}$-Oxide und deren Additionssalze mit einer anorganischen oder organischen, pharmazeutisch annehmbaren Säure.

2. Verbindung der Formel I nach Anspruch 1, nämlich N-(4-O-Desacetyl-5′-noranhydro-23-vinblastinoyl)-1-amino-2-methyl-propyl-phosphonsaurediethylester, in Form einer Mischung der Diastereoisomeren oder der reinen Isomeren und dessen Additionssalze mit einer anorganischen oder organischen, pharmazeutisch annehmbaren Säure.

3. Verbindung der Formel I nach Anspruch 1, nämlich (+)-N-($N_a$-Desformyl-4-O-desacetyl-23-vincristinoyl)-1-amino-2-methyl-propyl-phosphonsäurediethylester und dessen Additionssalze mit einer anorganischen oder organischen, pharmazeutisch annehmbaren Säure.

4. Verbindung der Formel I nach Anspruch 1, nämlich (+)-N-(4-O-Desacetyl-23-vincristinoyl)-1-amino-2-methyl-propyl-phosphonsäurediethylester und dessen Additionssalze mit einer anorganischen oder organischen, pharmazeutisch annehmbaren Säure.

5. Verbindung der Formel I nach Anspruch 1, nämlich (-)-N-(4-O-Desacetyl-23-vincristinoyl)-1-amino-2-methyl-propyl-phosphonsäurediethylester und dessen Additionssalze mit einer anorganischen oder organischen, pharmazeutisch annehmbaren Säure.

6. Verbindung der Formel I nach Anspruch 1, nämlich (+)-N-(4-O-Desacetyl-23-vincristinoyl)-1-amino-ethyl-phosphonsäurediethylester und dessen Additionssalze mit einer anorganischen oder organischen, pharmazeutisch annehmbaren Säure.

7. Verfahren zur Herstellung der Verbindungen der allgemeinen Formel I, **dadurch gekennzeichnet,** daß man
entweder
ein Amin der allgemeinen Formel II in Form des Racemats oder in optisch reiner Form:

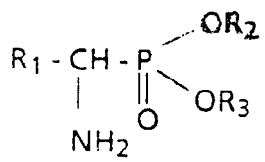

(II)

in der $R_1$, $R_2$ und $R_3$ die bezüglich der Formel I in Anspruch 1 angegebenen Bedeutungen besitzen, mit einer Verbindung der Formel III:

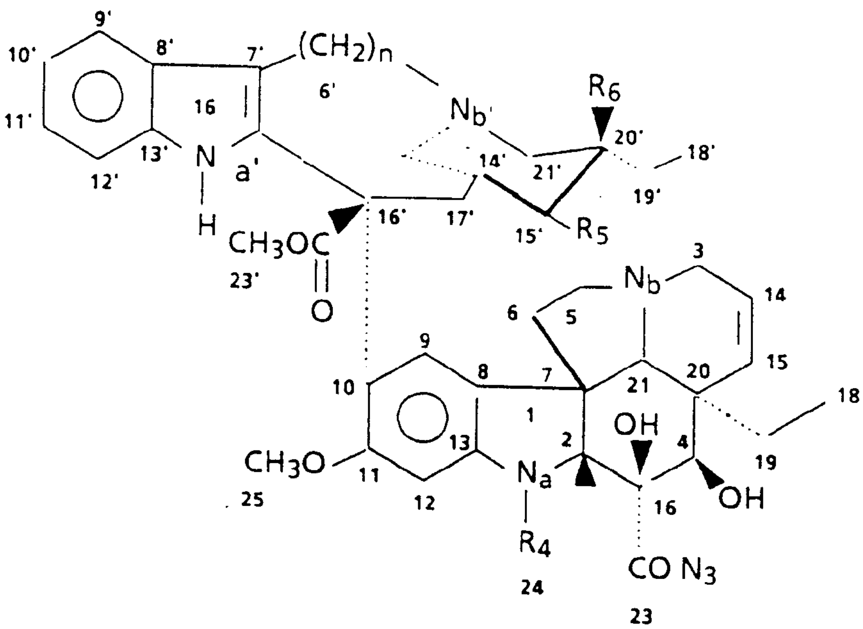

(III)

in der n, $R_5$ und $R_6$ die in Anspruch 1 bezüglich der allgemeinen Formel I angegebenen Bedeutungen besitzen und $R_4$ ein Wasserstoffatom oder eine Methylgruppe darstellt, umsetzt, so daß man die Verbindungen der Formel I nach Anspruch 1, worin n, $R_1$, $R_2$, $R_3$, $R_5$ und $R_6$ die oben angegebenen Bedeutungen besitzen und $R_4$ ein Wasserstoffatom oder eine Methylgruppe darstellt, in Form einer Mischung der Diastereoisomeren oder der reinen Isomeren erhält,
und dann
zur Bildung der Verbindungen der allgemeinen Formel I nach Anspruch 1, worin $R_4$ eine Formylgruppe darstellt,
die Verbindungen der Formel Ia:

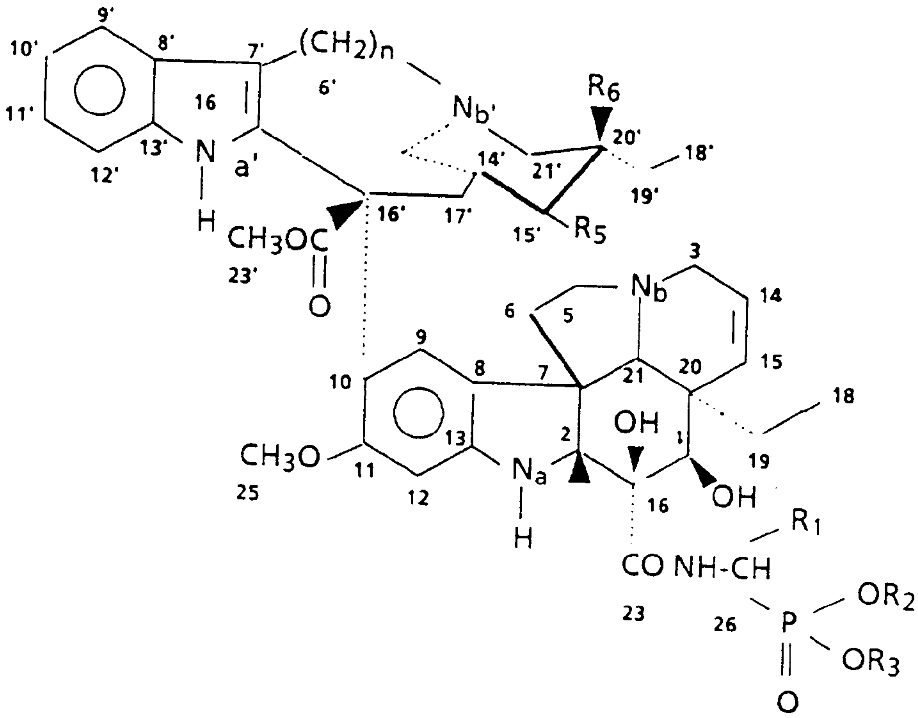

(Ia)

in Gegenwart von Essigsäureanhydrid mit Ameisensäure umsetzt,

<u>oder</u>

eine Verbindung der Formel IV in Form des Racemats oder in reiner optisch aktiver Form:

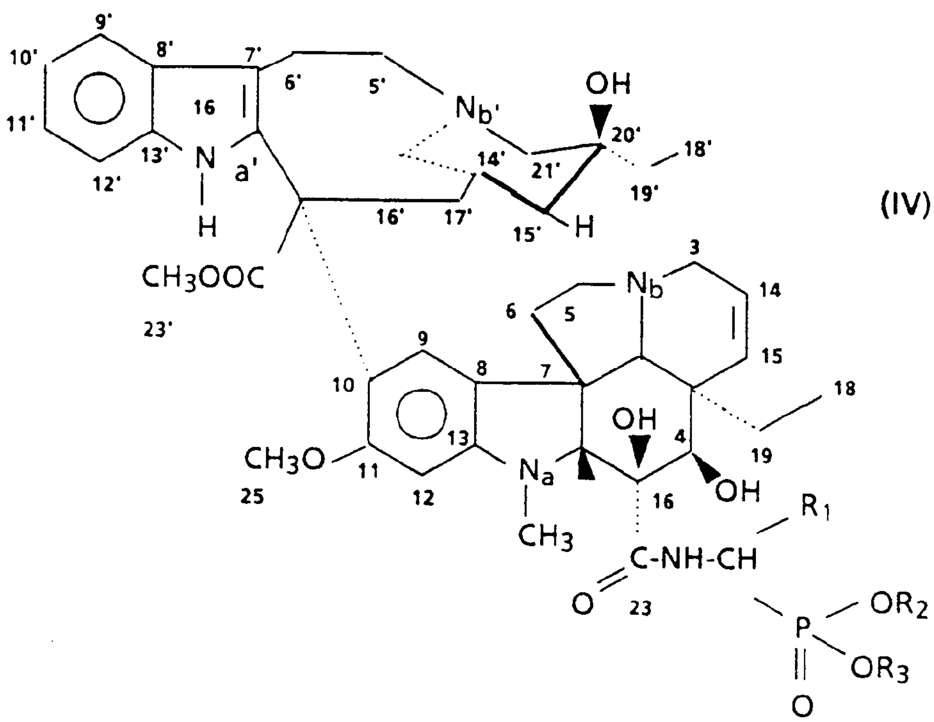

(IV)

in der $R_1$, $R_2$ und $R_3$ die oben angegebenen Bedeutungen besitzen, wie sie in Anspruch 1 bezüglich der Formel I angegeben worden sind,

in saurem Medium und in einem inerten Lösungsmittel bei einer Temperatur zwischen -40°C und -75°C mit Permanganationen umsetzt

zur Bildung der Verbindungen der allgemeinen Formel I nach Anspruch 1, worin n den Wert 2 be-

sitzt und $R_4$ eine Formylgruppe darstellt,
und anschließend
die Verbindungen der allgemeinen Formel I nach Anspruch 1 mit einer anorganischen oder organischen, pharmazeutisch annehmbaren Säure in ein Salz überführt,
oder
mit einem basischen organischen Lösungsmittel, welches mit Sauerstoff gesättigt ist, in die entsprechenden $N_{b'}$-Oxide umwandelt.

8. Pharmazeutische Zubereitung enthaltend als Wirkstoff eine Verbindung nach einem der Ansprüche 1 bis 6 in Kombination oder in Mischung mit einem inerten, nichttoxischen, pharmazeutisch annehmbaren Trägermaterial oder Bindemittel.

9. Pharmazeutische Zubereitung nach Anspruch 8 enthaltend den Wirkstoff in einer Dosis von 0,1 bis 100 mg.

10. Pharmazeutische Zubereitung nach den Ansprüchen 8 und 9 enthaltend als Wirkstoff mindestens eine Verbindung nach einem der Ansprüche 1 bis 4, geeignet für die Behandlung von neoplastischen Krankheiten an Lebewesen.

**Patentansprüche für folgenden Vertragsstaat : ES**

1. Verfahren zur Herstellung der Verbindungen der Formel I:

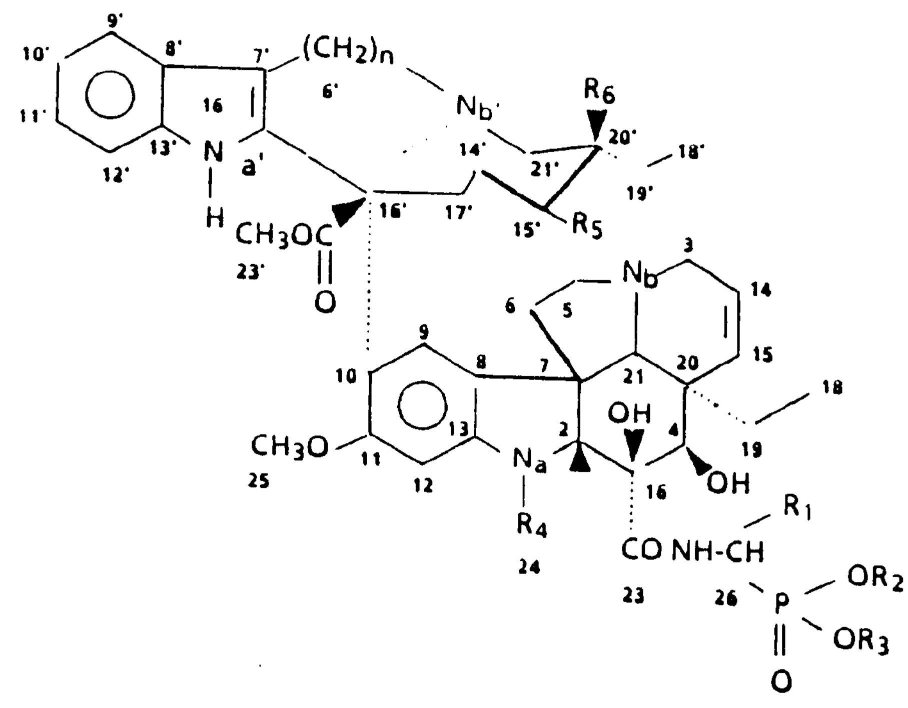

(I)

in der

- $R_1$ ein Wasserstoffatom, eine geradkettige oder verzweigte Alkylgruppe mit 1 bis 6 Kohlenstoffatomen, eine geradkettige oder verzweigte Alkenylgruppe mit 2 bis 6 Kohlenstoffatomen, eine Arylalkylgruppe mit 7 bis 10 Kohlenstoffatomen, die als Substituent am aromatischen Ring ein Halogenatom, eine Hydroxylgruppe oder eine Alkyl- oder Alkoxygruppe mit jeweils 1 bis 5 Kohlenstoffatomen tragen kann, eine Indol-2-yl-methylgruppe, eine Imidazol-4-yl-methylgruppe oder eine Alkoxycarbonylmethylgruppe mit 3 bis 11 Kohlenstoffatomen,
- $R_2$ und $R_3$, die gleichartig oder verschieden sein können, jeweils eine geradkettige oder verzweigte Alkylgruppe mit 1 bis 6 Kohlenstoffatomen,
- n 1 oder 2,
- $R_4$ ein Wasserstoffatom, eine Formylgruppe oder eine Methylgruppe mit der Maßgabe, daß $R_4$ keine Methylgruppe darstellt, wenn n den Wert 2 besitzt, und

- $R_5$ und $R_6$ entweder gemeinsam eine Doppelbindung oder $R_6$ ein Wasserstoff- atom und $R_6$ eine Hydroxylgruppe

bedeuten,
in Form einer Mischung der Diastereoisomeren oder der reinen Isomeren und deren $N_{b'}$-Oxide und von deren Additionssalzen mit einer anorganischen oder organischen, pharmazeutisch annehmbaren Säure, **dadurch gekennzeichnet,** daß man
entweder
ein Amin der allgemeinen Formel II in Form des Racemats oder in optisch reiner Form:

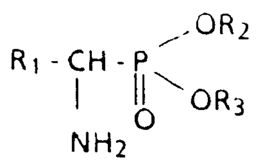

(II)

in der $R_1$, $R_2$ und $R_3$ die bezüglich der Formel I angegebenen Bedeutungen besitzen,
mit einer Verbindung der Formel III:

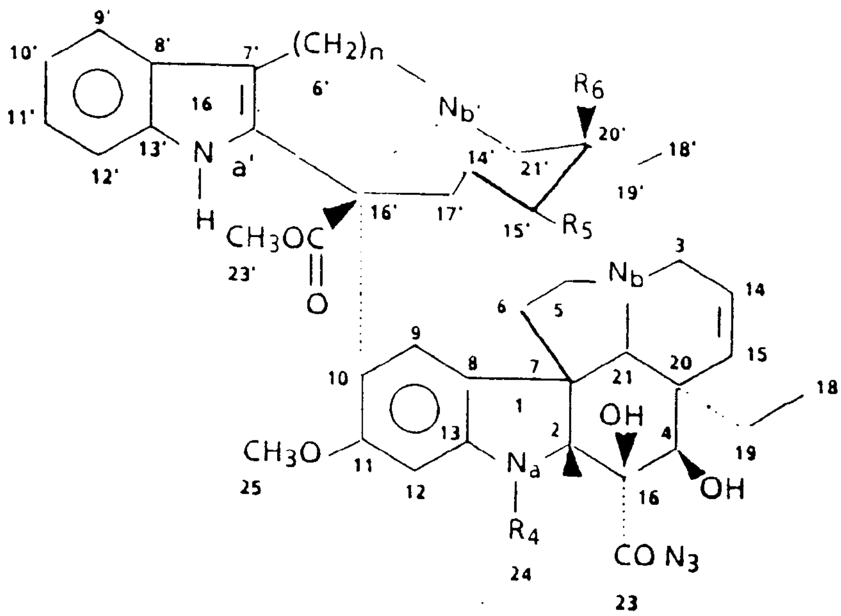

(III)

in der n, $R_5$ und $R_6$ die in Anspruch 1 bezüglich der allgemeinen Formel I angegebenen Bedeutungen besitzen und $R_4$ ein Wasserstoffatom oder eine Methylgruppe darstellt, umsetzt, so daß man die Verbindungen der Formel I, worin n, $R_1$, $R_2$, $R_3$, $R_6$ und $R_6$ die oben angegebenen Bedeutungen besitzen und $R_4$ ein Wasserstoffatom oder eine Methylgruppe darstellt, in Form einer Mischung der Diastereoisomeren oder der reinen Isomeren erhält,
und dann
zur Bildung der Verbindungen der allgemeinen Formel I, worin $R_4$ eine Formylgruppe darstellt,
die Verbindungen der Formel Ia:

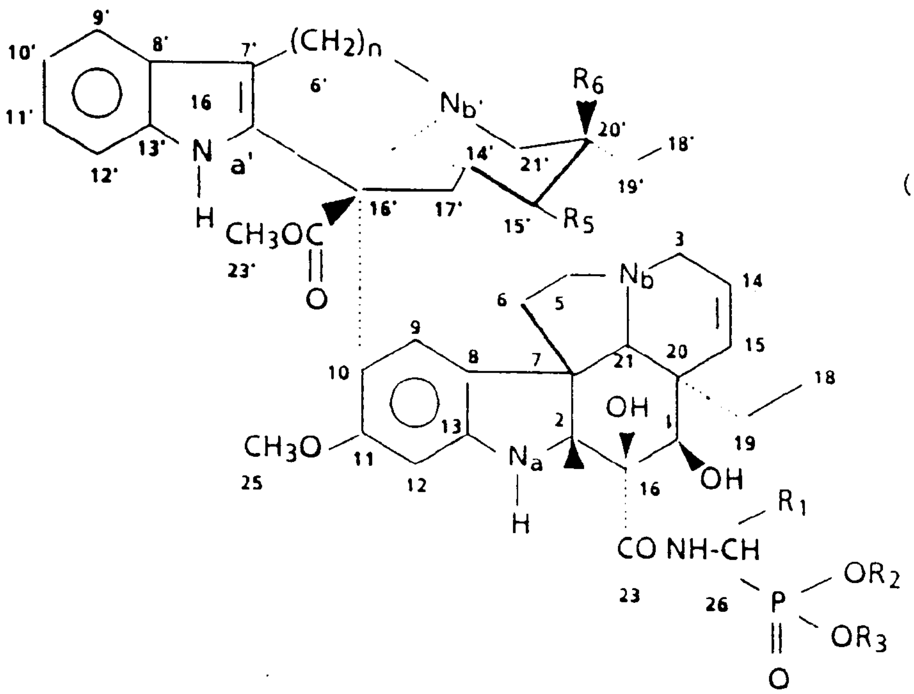

(Ia)

in Gegenwart von Essigsäureanhydrid mit Ameisensäure umsetzt,

oder

eine Verbindung der Formel IV in Form des Racemats oder in reiner optisch aktiver Form:

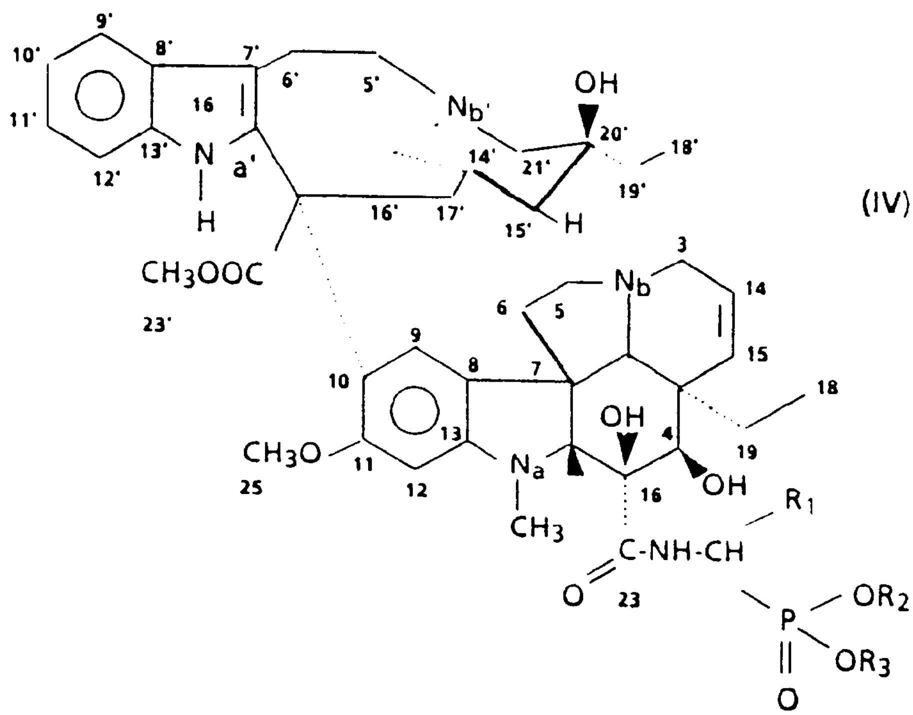

(IV)

in der $R_1$, $R_2$ und $R_3$ die oben angegebenen Bedeutungen besitzen, wie sie bezüglich der Formel I angegeben worden ist,

in saurem Medium und in einem inerten Lösungsmittel bei einer Temperatur zwischen -40°C und -75°C mit Permanganationen umsetzt

zur Bildung der Verbindungen der allgemeinen Formel I, worin n den Wert 2 besitzt und $R_4$ eine Formylgruppe darstellt,

und anschließend

die Verbindungen der allgemeinen Formel I nach Anspruch 1 mit einer anorganischen oder organischen, pharmazeutisch annehmbaren Säure in ein Salz überführt,
oder
mit einem basischen organischen Lösungsmittel, welches mit Sauerstoff gesättigt ist, in die entsprechenden $N_{b'}$-Oxide umwandelt.

2. Verbindung der Formel I nach Anspruch 1, nämlich N-(4-O-Desacetyl-5′-noranhydro-23-vinblastinoyl)-1-amino-2-methyl-propyl-phosphonsäurediethylester in Form einer Mischung der Diastereoisomeren oder der reinen Isomeren und deren Additionssalze mit einer anorganischen oder organischen, pharmazeutisch annehmbaren Säure, hergestellt nach dem Verfahren nach Anspruch 1 oder einem anderen chemisch äquivalenten Verfahren.

3. Verbindung der Formel I nach Anspruch 1, nämlich (+)-N-($N_a$-Desformyl-4-O-desacetyl-23-vincristinoyl)-1-amino-2-methyl-propyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

4. Verbindung der Formel I nach Anspruch 1, nämlich (+)-N-(4-O-Desacetyl-23-vincristinoyl)-1-amino-2-methyl-propyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

5. Verbindung der Formel I nach Anspruch 1, nämlich (-)-N-(4-O-Desacetyl-23-vincristinoyl)-1-amino-2-methyl-propyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

6. Verbindung der Formel I nach Anspruch 1, nämlich (+)-N-(4-O-Desacetyl-23-vincristinoyl)-1-amino-ethyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

**Patentansprüche für folgenden Vertragsstaat : GR**

1. Verfahren zur Herstellung der Verbindungen der Formel I:

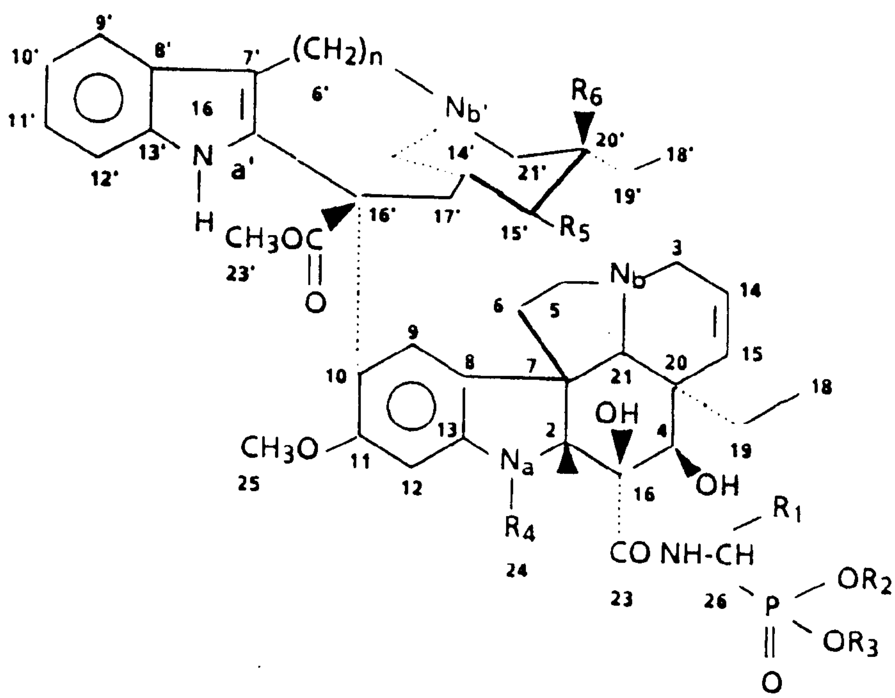

(I)

in der

- $R_1$ ein Wasserstoffatom, eine geradkettige oder verzweigte Alkylgruppe mit 1 bis 6 Kohlenstoffatomen, eine geradkettige oder verzweigte Alkenylgruppe mit 2 bis 6 Kohlenstoffatomen, eine Arylalkylgruppe mit 7 bis 10 Kohlenstoffatomen, die als Substituent am aromatischen Ring ein Halogenatom, eine Hydroxylgruppe oder eine Alkyl- oder Alkoxygruppe mit jeweils 1 bis 5 Kohlenstoffatomen tragen kann, eine Indol-2-yl-methylgruppe, eine Imidazol-4-yl-methylgruppe oder eine Alkoxycarbonylmethylgruppe mit 3 bis 11 Kohlenstoffatomen,
- $R_2$ und $R_3$, die gleichartig oder verschieden sein können, jeweils eine geradkettige oder verzweigte Alkylgruppe mit 1 bis 6 Kohlenstoffatomen,
- n 1 oder 2,
- $R_4$ ein Wasserstoffatom, eine Formylgruppe oder eine Methylgruppe mit der Maßgabe, daß $R_4$ keine Methylgruppe darstellt. wenn n den Wert 2 besitzt, und
- $R_5$ und $R_6$ entweder gemeinsam eine Doppelbindung oder $R_5$ ein Wasserstoffatom und $R_6$ eine Hydroxylgruppe bedeuten,

in Form einer Mischung der Diastereoisomeren oder der reinen Isomeren und deren $N_{b'}$-Oxide und deren Additionssalze mit einer anorganischen oder organischen, pharmazeutisch annehmbaren Säure, **dadurch gekennzeichnet,** daß man

entweder

ein Amin der allgemeinen Formel II in Form des Racemats oder in optisch reiner Form:

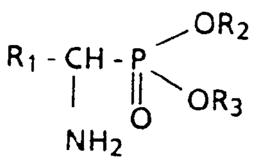

(II)

in der $R_1$, $R_2$ und $R_3$ die bezüglich der Formel I angegebenen Bedeutungen besitzen, mit einer Verbindung der Formel III:

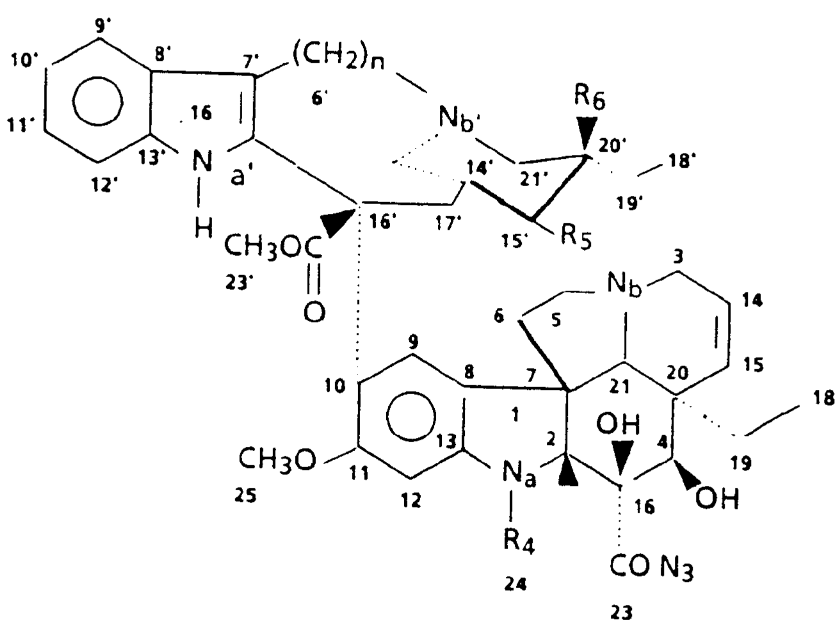

(III)

in der n, $R_5$ und $R_6$ die in Anspruch 1 bezüglich der allgemeinen Formel I angegebenen Bedeutungen besitzen und $R_4$ ein Wasserstoffatom oder eine Methylgruppe darstellt, umsetzt, so daß man die Verbindungen der Formel I, worin n, $R_1$, $R_2$ $R_3$, $R_5$ und $R_6$ die oben angegebenen Bedeutungen besitzen und $R_4$ ein Wasserstoffatom oder eine Methylgruppe darstellt, in Form einer Mischung der Diastereoisomeren oder der reinen Isomeren erhält,

und dann

zur Bildung der Verbindungen der allgemeinen Formel I, worin $R_4$ eine Formylgruppe darstellt,

die Verbindungen der Formel Ia:

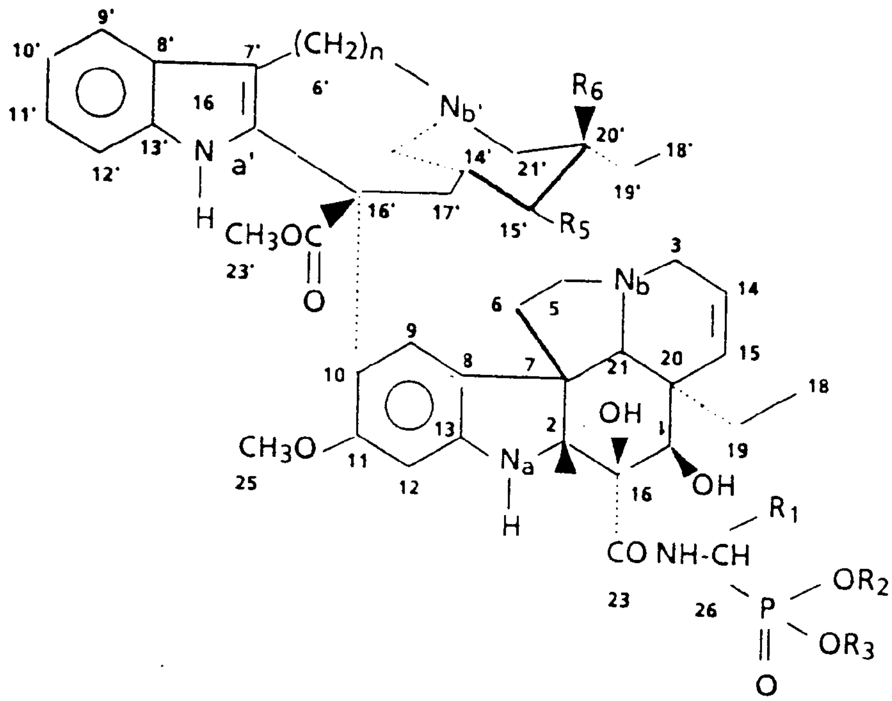

(Ia)

in Gegenwart von Essigsäureanhydrid mit Ameisensäure umsetzt,

<u>oder</u>

eine Verbindung der Formel IV in Form des Racemats oder in reiner optisch aktiver Form:

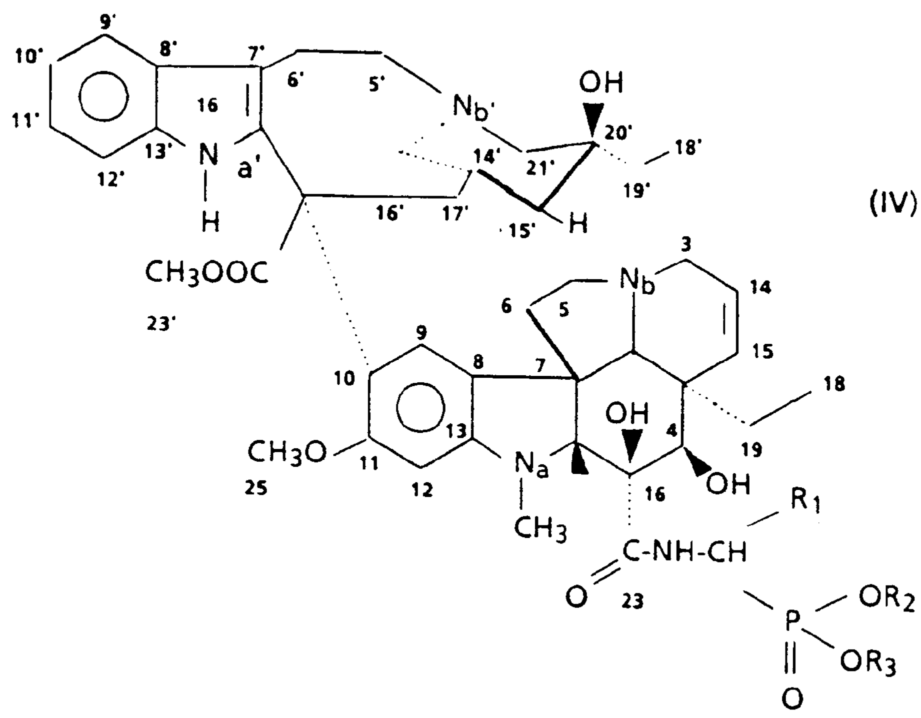

(IV)

in der $R_1$, $R_2$ und $R_3$ die oben angegebenen Bedeutungen besitzen, wie sie bezüglich der Formel I angegeben worden ist,

in saurem Medium und in einem inerten Lösungsmittel bei einer Temperatur zwischen -40°C und -75°C mit Permanganationen umsetzt

zur Bildung der Verbindungen der allgemeinen Formel I, worin n den Wert 2 besitzt und $R_4$ eine Formylgruppe darstellt,

und anschließend

die Verbindungen der allgemeinen Formel I nach Anspruch 1 mit einer anorganischen oder organischen, pharmazeutisch annehmbaren Säure in ein Salz überführt,

oder

mit einem basischen organischen Lösungsmittel, welches mit Sauerstoff gesättigt ist, in die entsprechenden $N_{b'}$-Oxide umwandelt.

2. Verbindung der Formel I nach Anspruch 1, nämlich N-(4-O-Desacetyl-5′-noranhydro-23-vinblastinoyl)-1-amino-2-methyl-propyl-phosphonsäurediethylester in Form einer Mischung der Diastereoisomeren oder der reinen Isomeren und deren Additionssalze mit einer anorganischen oder organischen, pharmazeutisch annehmbaren Säure, hergestellt nach dem Verfahren nach Anspruch 1 oder einem anderen chemisch äquivalenten Verfahren.

3. Verbindung der Formel I nach Anspruch 1, nämlich (+)-N-($N_a$-Desformyl-4-O-desacetyl-23-vincristinoyl)-1-amino-2-methyl-propyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

4. Verbindung der Formel I nach Anspruch 1, nämlich (+)-N-(4-O-Desacetyl-23-vincristinoyl)-1-amino2-methyl-propyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

5. Verbindung der Formel I nach Anspruch 1, nämlich (-)-N-(4-O-Desacetyl-23-vincristinoyl)-1-amino-2-methyl-propyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

6. Verbindung der Formel I nach Anspruch 1, nämlich (+)-N-(4-O-Desacetyl- 23-vincristinoyl)-1-amino-ethyl-phosphonsäurediethylester und dessen Additionssalze mit einer anorganischen oder organischen, pharmazeutisch annehmbaren Säure, hergestellt nach dem Verfahren nach Anspruch 1 oder einem anderen chemisch äquivalenten Verfahren.

## Claims

### Claims for the following Contracting States : AT, BE, CH, DE, DK, FR, GB, IT, LI, LU, NL, SE

1. Compounds of formula I :

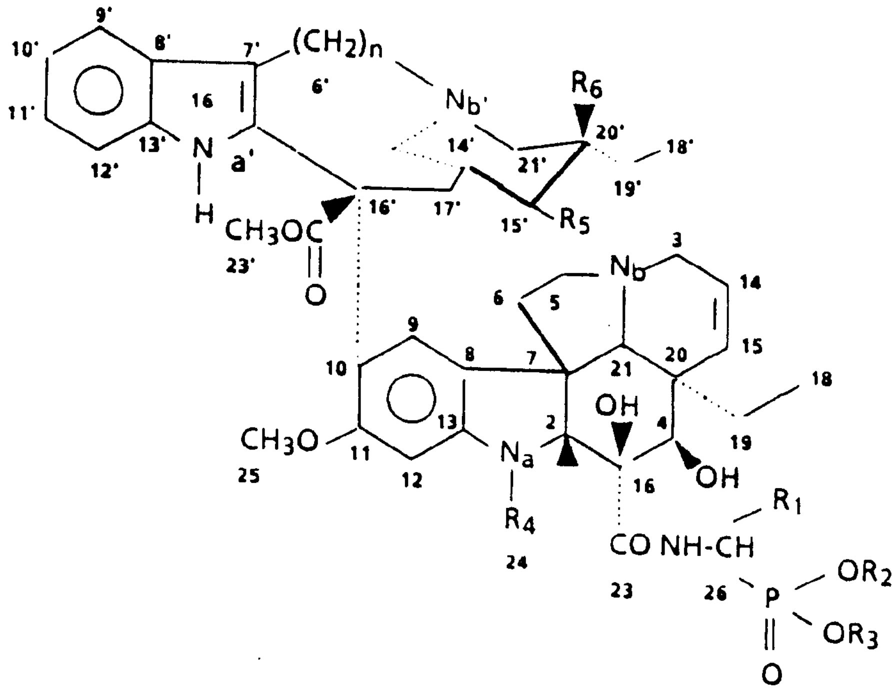

(I)

wherein :

- $R_1$ represents a hydrogen atom, a straight-chain or branched alkyl radical containing from 1 to 6 carbon atoms, a straight-chain or branched alkenyl radical containing from 2 to 6 carbon atoms, an aralkyl radical containing from 7 to 10 carbon atoms and optionally carrying as substituent at the aromatic ring a halogen atom, a hydroxy radical or an alkyl or alkoxy radical each containing from 1 to 5 carbon atoms, or $R_1$ represents an indol-2-ylmethyl radical, an imidazol-4-ylmethyl radical, or an alkoxy-carbonylmethyl radical containing from 3 to 11 carbon atoms,
- $R_2$ and $R_3$ are the same or different and each represents a straight-chain or branched alkyl radical containing from 1 to 6 carbon atoms,
- n is 1 or 2,
- $R_4$ represents a hydrogen atom, a formyl radical or a methyl radical, with the proviso that $R_4$ is not a methyl radical when n is 2,
  and
- $R_5$ and $R_6$ either together form a double bond or $R_5$ represents a hydrogen atom and $R_6$ represents a hydroxy radical,

in the form of a mixture of diastereoisomers or in the form of pure isomers, their $N_{b'}$-oxides and their addition salts with a pharmaceutically acceptable mineral or organic acid.

2. Diethyl N-(4-O-deacetyl-5′-noranhydro-23-vinblastinoyl)-1-amino-2-methylpropylphosphonate, a compound corresponding to formula I according to claim 1, in the form of a mixture of diastereoisomers or in the form of pure isomers, and its addition salts with a pharmaceutically acceptable mineral or organic acid.

3. (+)-Diethyl N-($N_a$-deformyl-4-O-deacetyl-23-vincristinoyl)-1-amino-2-methylpropylphosphonate, a compound corresponding to formula I according to claim 1, and its addition salts with a pharmaceutically acceptable mineral or organic acid.

4. (+)-Diethyl N-(4-O-deacetyl-23-vincristinoyl)-1-amino-2-methylpropylphosphonate, a compound corresponding to formula I according to claim 1, and its addition salts with a pharmaceutically acceptable mineral or organic acid.

5. (-)-Diethyl N-(4-O-deacetyl-23-vincristinoyl)-1-amino-2-methylpropylphosphonate, a compound corresponding to formula I according to claim 1, and its addition salts with a pharmaceutically acceptable mineral or organic acid.

**6.** (+)-Diethyl N-(4-O-deacetyl-23-vincristinoyl)-1-aminoethylphosphonate, a compound corresponding to formula I according to claim 1, and its addition salts with a pharmaceutically acceptable mineral or organic acid.

**7.** A process for the preparation of compounds of the general formula I, characterised in that
either
an amine, in racemic or optically pure form, of the general formula II :

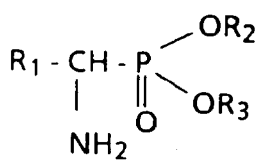

(II)

in which the definitions of $R_1$, $R_2$ and $R_3$ remain the same as those given above for the general formula I according to claim 1, is reacted
with a compound of formula III

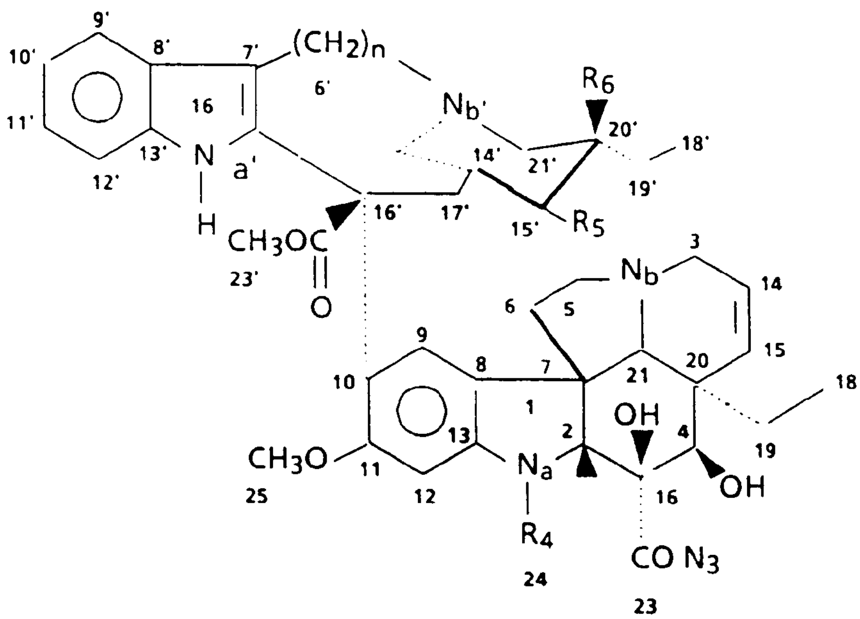

(III)

wherein n, $R_5$ and $R_5$ are as defined above for the general formula I according to claim 1, and $R_4$ represents a hydrogen atom or a methyl radical, to yield in the form of a mixture of diastereoisomers or in the form of pure isomers, the compounds of the general formula I according to claim 1 wherein n, $R_1$, $R_2$, $R_3$, $R_5$ and $R_5$ are as defined above and $R_4$ represents a hydrogen atom or a methyl radical,
and, to produce compounds of the general formula I according to claim 1 wherein $R_4$ represents a formyl radical,
the compounds of formula Ia :

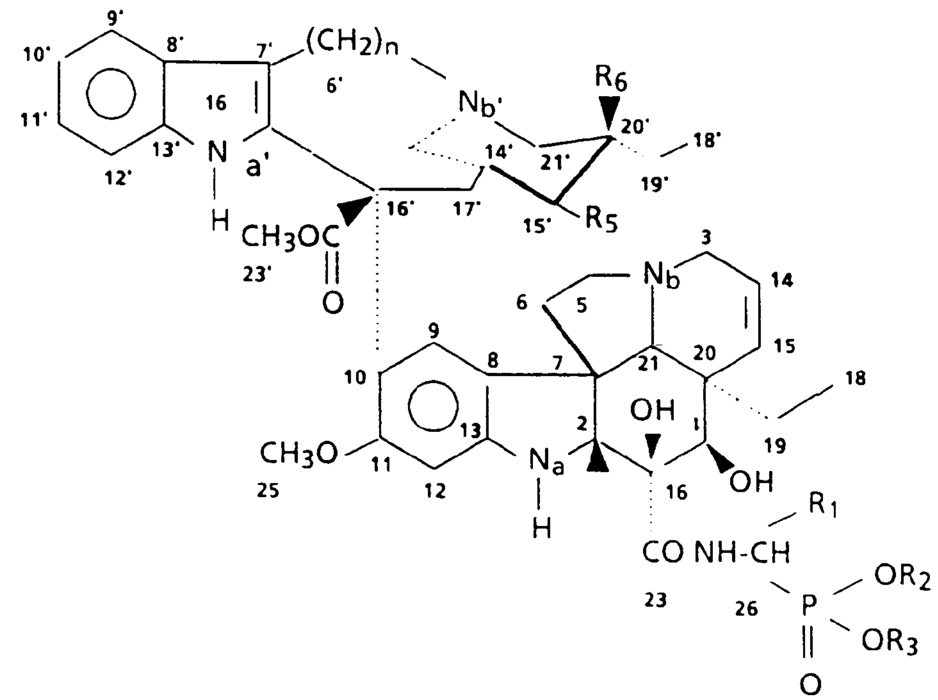

(Ia)

are subjected to the action of formic acid in the presence of acetic anhydride,

or

a compound in racemic or pure optically active form of formula IV :

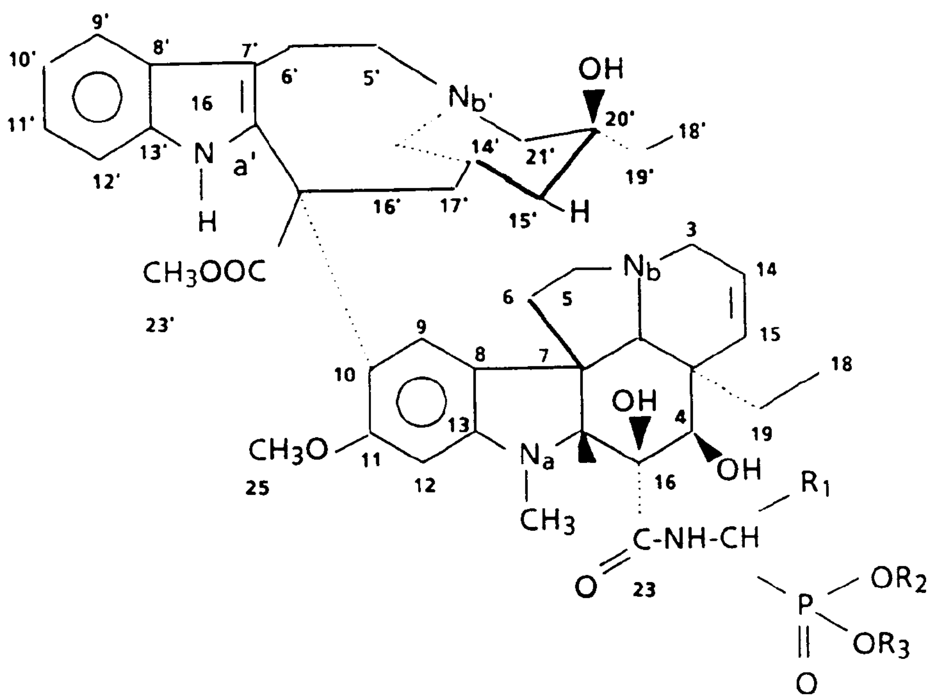

(IV)

in which the definitions of $R_1$, $R_2$ and $R_3$ remain the same as those given above for formula I according to claim 1,

is treated with the permanganate ion in acidic medium in an inert solvent, at a temperature of from -40°C to -75°C,

to yield compounds of the general formula I according to claim 1 wherein n is 2 and $R_4$ represents a formyl radical,

and then,
the compounds of the general formula I according to claim 1 are converted into salts with a pharmaceutically acceptable mineral or organic acid,
or
are converted into the corresponding $N_{b'}$-oxides using a basic organic solvent saturated with oxygen.

**8.** Pharmaceutical composition comprising as active ingredient a compound according to any one of claims 1 to 6, in association or mixture with a pharmaceutically acceptable, inert, non-toxic excipient or carrier.

**9.** Pharmaceutical composition according to claim 8, containing the active ingredient in an amount of from 0.1 to 100 mg.

**10.** Pharmaceutical composition according to claim 8 or 9 containing as active ingredient at least one compound according to any one of claims 1 to 4 which can be used for the treatment of neoplastic disorders in human beings.

**Claims for the following Contracting State : ES**

**1.** Process for the preparation of compounds of formula I:

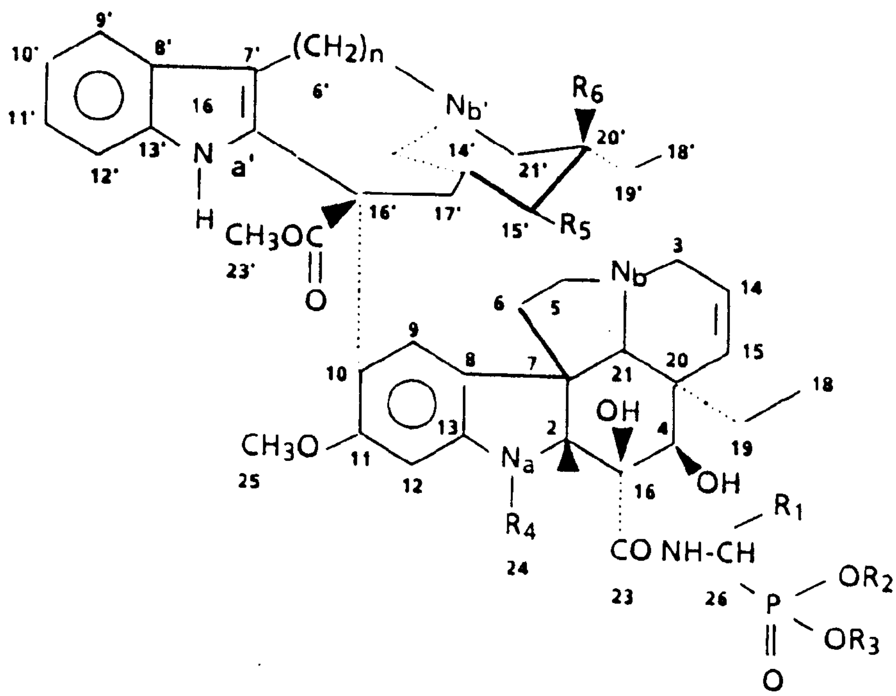

(I)

wherein :
- $R_1$ represents a hydrogen atom, a straight-chain or branched alkyl radical containing from 1 to 6 carbon atoms, a straight-chain or branched alkenyl radical containing from 2 to 6 carbon atoms, an aralkyl radical containing from 7 to 10 carbon atoms and optionally carrying as substituent at the aromatic ring a halogen atom, a hydroxy radical or an alkyl or alkoxy radical each containing from 1 to 5 carbon atoms, or $R_1$ represents an indol-2-ylmethyl radical, an imidazol-4-ylmethyl radical, or an alkoxycarbonylmethyl radical containing from 3 to 11 carbon atoms,
- $R_2$ and $R_3$ are the same or different and each represents a straight-chain or branched alkyl radical containing from 1 to 6 carbon atoms,
- n is 1 or 2,
- $R_4$ represents a hydrogen atom, a formyl radical or a methyl radical, with the proviso that $R_4$ is not a methyl radical when n is 2,
  and
- $R_5$ and $R_6$ either together form a double bond or $R_5$ represents a hydrogen atom and $R_6$ represents a hydroxy radical,

in the form of a mixture of diastereoisomers or in the form of pure isomers, their $N_{b'}$-oxides and their addition salts with a pharmaceutically acceptable mineral or organic acid,
characterised in that
<u>either</u>

an amine, in racemic or optically pure form, of the general formula II :

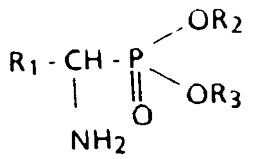

(II),

in which the definitions of $R_1$, $R_2$ and $R_3$ remain the same as those given above for the general formula I, is reacted
with a compound of formula III

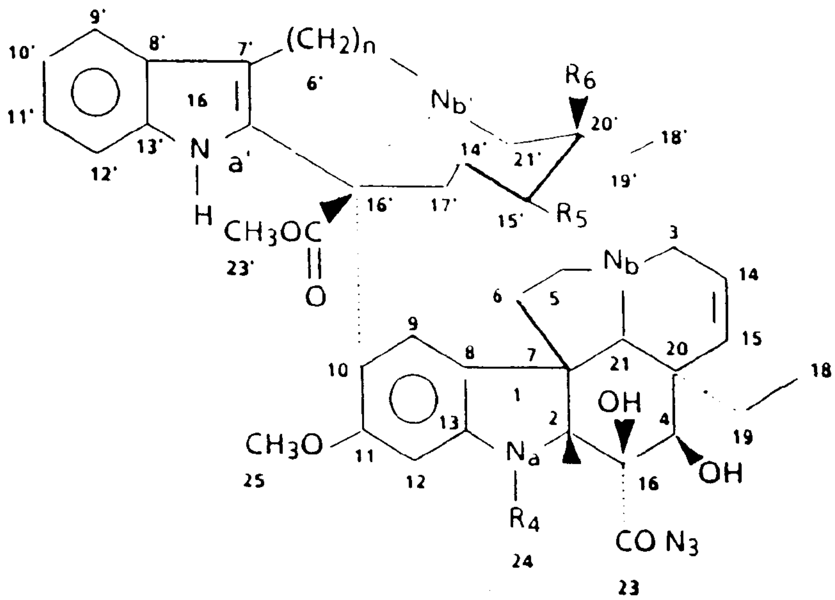

(III)

wherein n, $R_5$ and $R_6$ are as defined above for the general formula I, and $R_4$ represents a hydrogen atom or a methyl radical, to yield in the form of a mixture of diastereoisomers or in the form of pure isomers, the compounds of the general formula I wherein n, $R_1$, $R_2$, $R_3$, $R_6$ and $R_6$ are as defined above and $R_4$ represents a hydrogen atom or a methyl radical,
and, to produce compounds of the general formula I wherein $R_4$ represents a formyl radical,
the compounds of formula Ia :

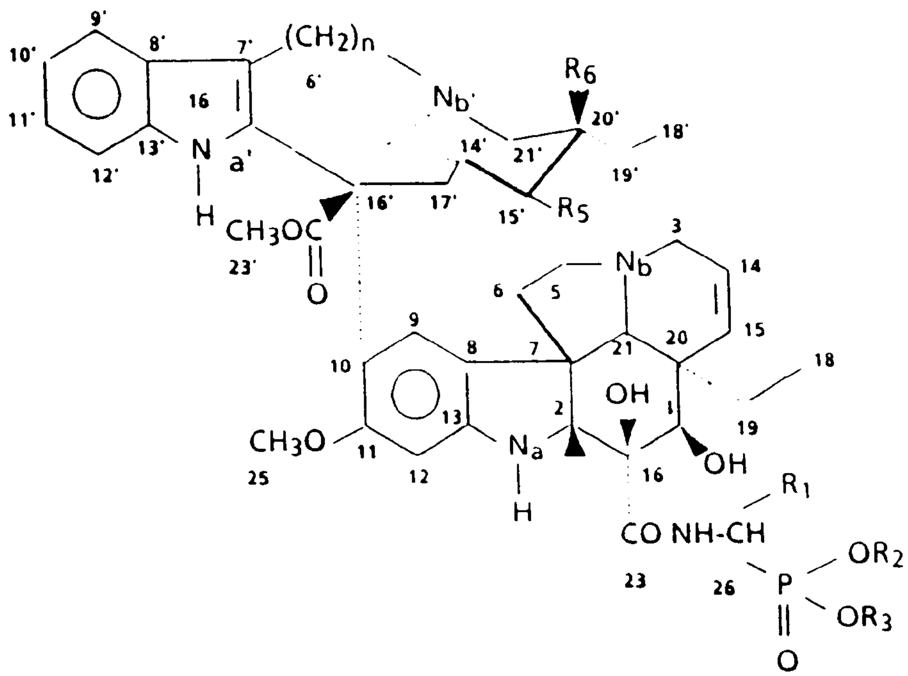

(Ia)

are subjected to the action of formic acid in the presence of acetic anhydride,

or

a compound in racemic or pure optically active form of formula IV :

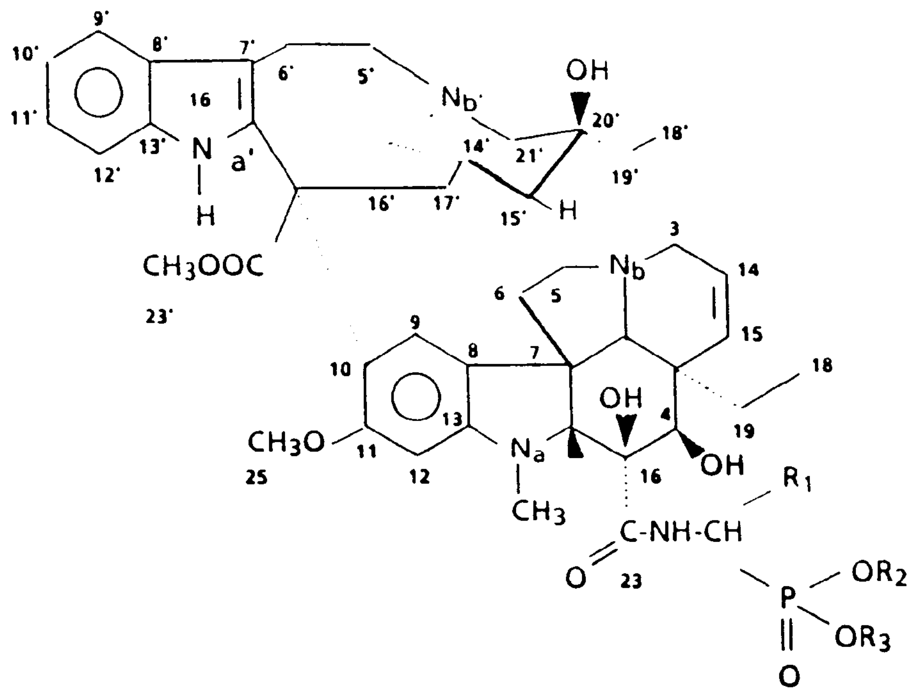

(IV)

in which the definitions of $R_1$, $R_2$ and $R_3$ remain the same as those given above for formula I,

is treated with the permanganate ion in acidic medium in an inert solvent, at a temperature of from -40°C to -75°C,

to yield compounds of the general formula I wherein n is 2 and $R_4$ represents a formyl radical,

and then,

the compounds of the general formula I are converted into salts with a pharmaceutically acceptable mineral or organic acid,

or

are converted into the corresponding $N_{b'}$-oxides using a basic organic solvent saturated with oxygen.

2. Diethyl N-(4-O-deacetyl-5′-noranhydro-23-vinblastinoyl)-1-amino-2-methylpropylphosphonate, a compound corresponding to formula I according to claim 1, in the form of a mixture of diastereoisomers or in the form of pure isomers, and its addition salts with a pharmaceutically acceptable mineral or organic acid, when prepared in accordance with the process of claim 1 or another chemically equivalent process.

3. (+)-Diethyl N-($N_a$-deformyl-4-O-deacetyl-23-vincristinoyl)-1-amino-2-methylpropylphosphonate, a compound corresponding to formula I according to claim 1, and its addition salts with a pharmaceutically acceptable mineral or organic acid, when prepared in accordance with the process of claim 1 or another chemically equivalent process.

4. (+)-Diethyl N-(4-O-deacetyl-23-vincristinoyl)-1-amino-2-methylpropylphosphonate, a compound corresponding to formula I according to claim 1, and its addition salts with a pharmaceutically acceptable mineral or organic acid, when prepared in accordance with the process of claim 1 or another chemically equivalent process.

5. (-)-Diethyl N-(4-O-deacetyl-23-vincristinoyl)-1-amino-2-methylpropylphosphonate, a compound corresponding to formula I according to claim 1, and its addition salts with a pharmaceutically acceptable mineral or organic acid, when prepared in accordance with the process of claim 1 or another chemically equivalent process.

6. (+)-Diethyl N-(4-O-deacetyl-23-vincristinoyl)-1-aminoethylphosphonate, a compound corresponding to formula I according to claim 1, and its addition salts with a pharmaceutically acceptable mineral or organic acid, when prepared in accordance with the process of claim 1 or another chemically equivalent process.

**Claims for the following Contracting State : GR**

1. Process for the preparation of compounds of formula I:

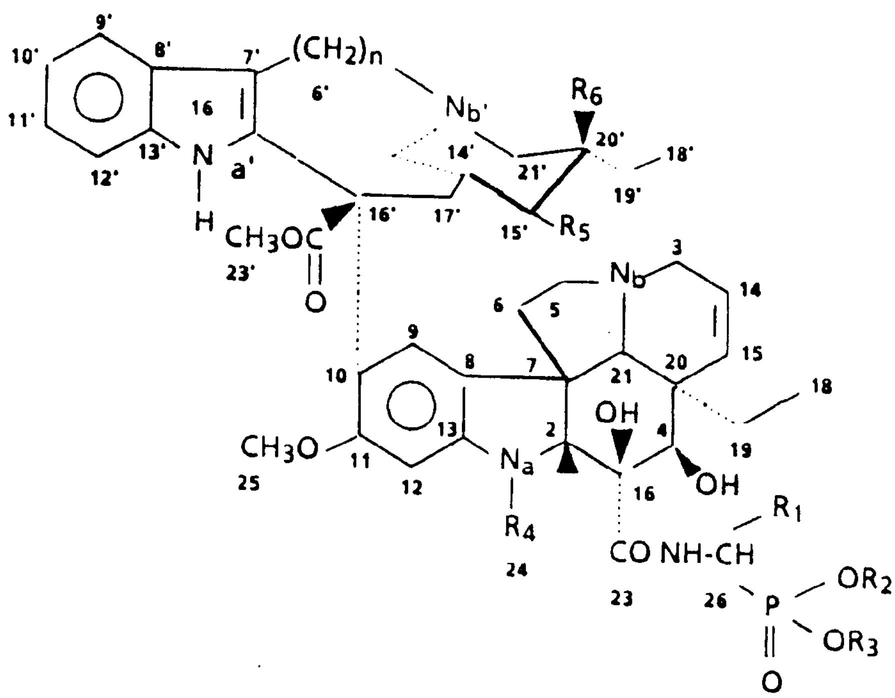

(I)

wherein :

- $R_1$ represents a hydrogen atom, a straight-chain or branched alkyl radical containing from 1 to 6 carbon atoms, a straight-chain or branched alkenyl radical containing from 2 to 6 carbon atoms, an aralkyl radical containing from 7 to 10 carbon atoms and optionally carrying as substituent at the aromatic ring a halogen atom, a hydroxy radical or an alkyl or alkoxy radical each containing from 1 to 5 carbon atoms, or $R_1$ represents an indol-2-ylmethyl radical, an imidazol-4-ylmethyl radical, or an alkoxycarbonylmethyl radical containing from 3 to 11 carbon atoms,

- $R_2$ and $R_3$ are the same or different and each represents a straight-chain or branched alkyl radical containing from 1 to 6 carbon atoms,
- n is 1 or 2,
- $R_4$ represents a hydrogen atom, a formyl radical or a methyl radical, with the proviso that $R_4$ is not a methyl radical when n is 2,
  and
- $R_5$ and $R_6$ either together form a double bond or $R_5$ represents a hydrogen atom and $R_6$ represents a hydroxy radical,

in the form of a mixture of diastereoisomers or in the form of pure isomers, their $N_{b'}$-oxides and their addition salts with a pharmaceutically acceptable mineral or organic acid,
characterised in that
either
an amine, in racemic or optically pure form, of the general formula II :

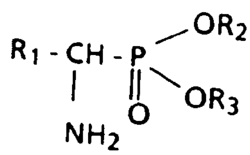

(II),

in which the definitions of $R_1$, $R_2$ and $R_3$ remain the same as those given above for the general formula I, is reacted
with a compound of formula III

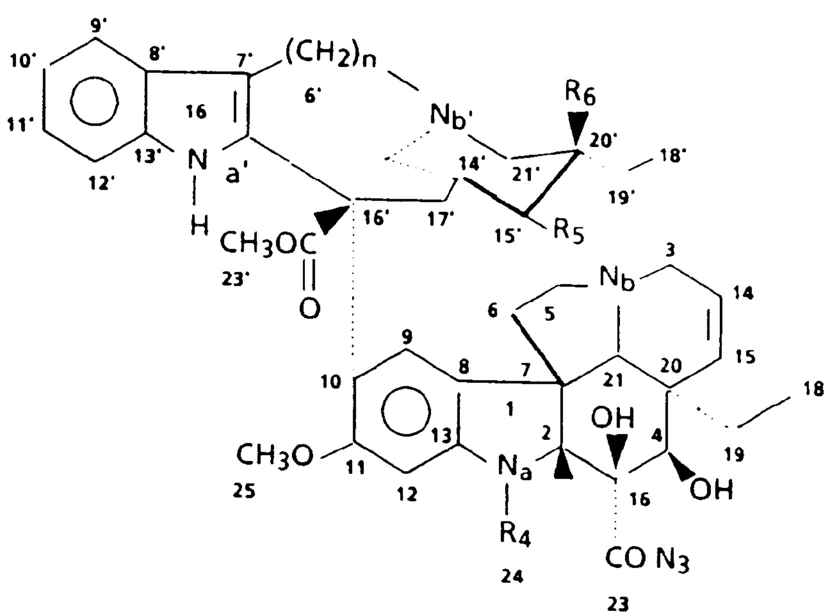

(III)

wherein n, $R_5$ and $R_5$ are as defined above for the general formula I, and $R_4$ represents a hydrogen atom or a methyl radical, to yield in the form of a mixture of diastereoisomers or in the form of pure isomers, the compounds of the general formula I wherein n, $R_1$, $R_2$, $R_3$, $R_5$ and $R_5$ are as defined above and $R_4$ represents a hydrogen atom or a methyl radical,
and, to produce compounds of the general formula I wherein $R_4$ represents a formyl radical,
the compounds of formula Ia :

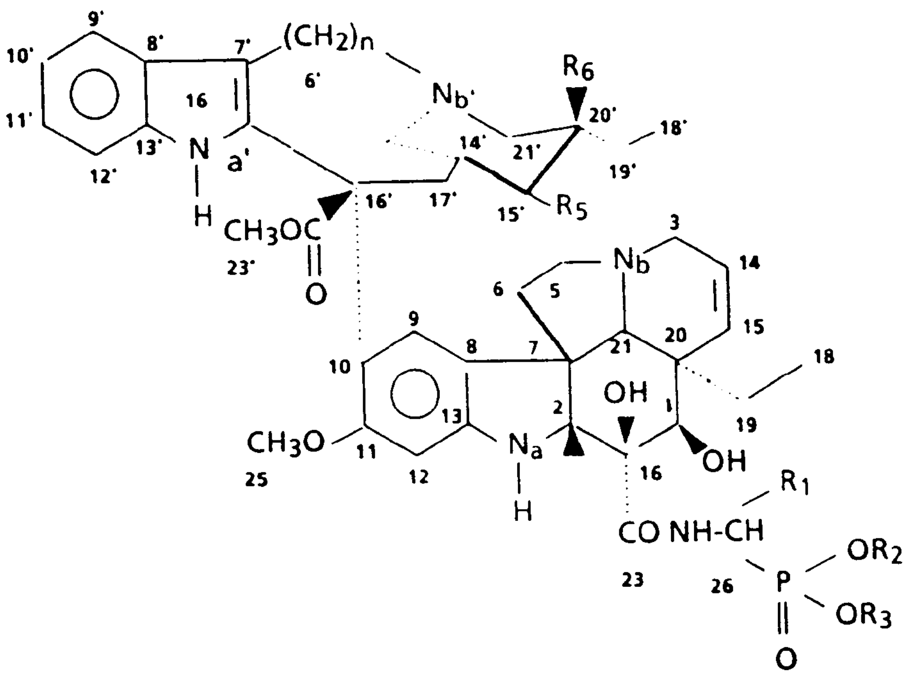

(Ia)

are subjected to the action of formic acid in the presence of acetic anhydride,

or

a compound in racemic or pure optically active form of formula IV :

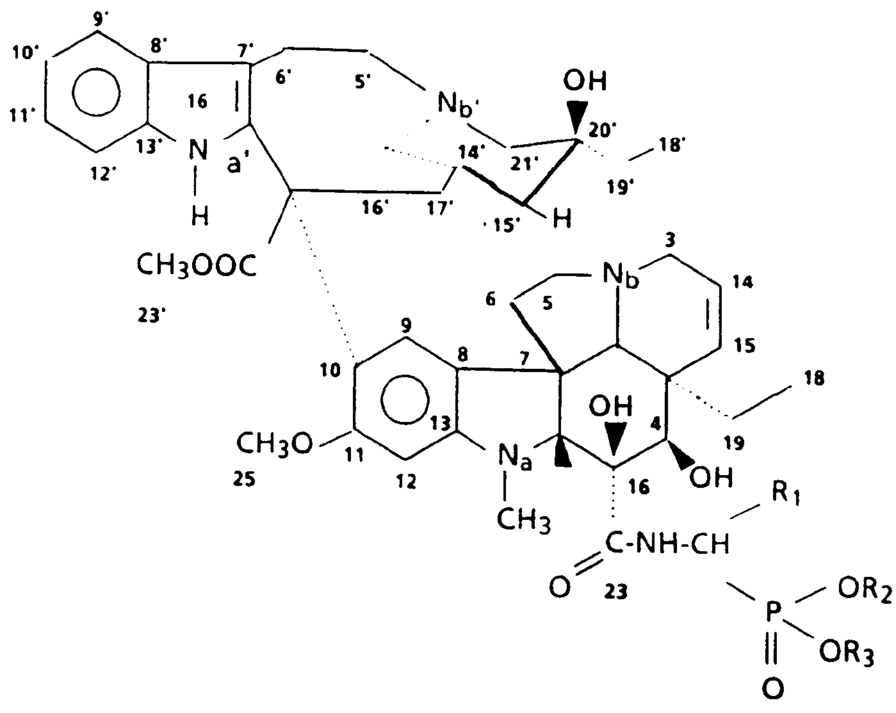

(IV)

in which the definitions of $R_1$, $R_2$ and $R_3$ remain the same as those given above for formula I,

is treated with the permanganate ion in acidic medium in an inert solvent, at a temperature of from -40°C to -75°C,

to yield compounds of the general formula I wherein n is 2 and $R_4$ represents a formyl radical,

and then,

the compounds of the general formula I are converted into salts with a pharmaceutically acceptable mineral or organic acid,

or
are converted into the corresponding $N_{b'}$-oxides using a basic organic solvent saturated with oxygen.

**2.** Diethyl N-(4-O-deacetyl-5′-noranhydro-23-vinblastinoyl)-1-amino-2-methylpropylphosphonate, a compound corresponding to formula I according to claim 1, in the form of a mixture of diastereoisomers or in the form of pure isomers, and its addition salts with a pharmaceutically acceptable mineral or organic acid, when prepared in accordance with the process of claim 1 or another chemically equivalent process.

**3.** (+)-Diethyl N-($N_a$-deformyl-4-O-deacetyl-23-vincristinoyl)-1-amino-2-methylpropylphosphonate, a compound corresponding to formula I according to claim 1, and its addition salts with a pharmaceutically acceptable mineral or organic acid, when prepared in accordance with the process of claim 1 or another chemically equivalent process.

**4.** (+)-Diethyl N-(4-O-deacetyl-23-vincristinoyl)-1-amino-2-methylpropylphosphonate, a compound corresponding to formula I according to claim 1, and its addition salts with a pharmaceutically acceptable mineral or organic acid, when prepared in accordance with the process of claim 1 or another chemically equivalent process.

**5.** (-)-Diethyl N-(4-O-deacetyl-23-vincristinoyl)-1-amino-2-methylpropylphosphonate, a compound corresponding to formula I according to claim 1, and its addition salts with a pharmaceutically acceptable mineral or organic acid, when prepared in accordance with the process of claim 1 or another chemically equivalent process.

**6.** (+)-Diethyl N-(4-O-deacetyl-23-vincristinoyl)-1-aminoethylphosphonate, a compound corresponding to formula I according to claim 1, and its addition salts with a pharmaceutically acceptable mineral or organic acid, when prepared in accordance with the process of claim 1 or another chemically equivalent process.